# EUROPÄISCHE PATENTSCHRIFT

(11) **EP 3 122 720 B1**
(45) Veröffentlichungstag und Bekanntmachung des Hinweises auf die Patenterteilung: **13.06.2018**
(21) Anmeldenummer: 15711761.5
(22) Anmeldetag: 24.03.2015
(51) Int. Cl.: C07C 263/10, C07C 265/14

(54) **VERFAHREN ZUM BETREIBEN EINER GASPHASENPHOSGENIERUNGSANLAGE**
METHOD FOR OPERATING A GAS PHASE PHOSGENATION SYSTEM
PROCÉDÉ DE FONCTIONNEMENT D'UNE INSTALLATION DE PHOSGÉNATION EN PHASE GAZEUSE

(30) Priorität: 27.03.2014 EP 14162002
(43) Veröffentlichungstag der Anmeldung: 01.02.2017
(73) Patentinhaber: Covestro Deutschland AG, 51373 Leverkusen (DE)
(72) Erfinder: KNAUF, Thomas, 41542 Dormagen (DE); LORENZ, Wolfgang, 41540 Dormagen (DE); STEFFENS, Friedhelm, 51373 Leverkusen (DE); BRUNS, Rainer, 51467 Bergisch Gladbach (DE); TAUBE, Wolfgang, 41470 Neuss (DE)
(74) Vertreter: Levpat
(86) Internationale Anmeldenummer: PCT/EP2015/056216
(87) Internationale Veröffentlichungsnummer: WO 2015/144682

(56) Entgegenhaltungen:
- WO-A1-2013/029918

## Beschreibung

Die Erfindung betrifft ein Verfahren zum Betreiben einer Gasphasenphosgenierungsanlage (**100**) zur Umsetzung eines Amins (**2**) mit Phosgen (**1**) zum korrespondierenden Isocyanat (**4**), bei dem zur Außerbetriebnahme der Gasphasenphosgenierungsanlage (**100**) zunächst der Aminstrom abgestellt und der Phosgenstrom noch aufrechterhalten wird. Frühestens nach Verstreichen eines Zeitraums, welcher der 10-fachen, bevorzugt der 30-fachen, besonders bevorzugt der 60-fachen, ganz besonders bevorzugt der 90-fachen Verweilzeit von Phosgen (**1**) in den wesentlichen Teilen der Gasphasenphosgenierungsanlage (**100**) im Regelbetrieb derselben entspricht, gerechnet ab dem Zeitpunkt, ab dem der Aminzufluss vollständig abgestellt wurde, wird auch die Phosgenzufuhr abgestellt. Durch die Amin- und Phosgenzuführungseinrichtungen sowie durch alle anderen wesentlichen Teile der Gasphasenphosgenierungsanlage (**100**) wird während der Außerbetriebnahme ein Inertgasstrom (**30**) aufrechterhalten.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen. Die Verfahrensführung in der Gasphase, üblicherweise als Gasphasenphosgenierung bezeichnet, zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind. Vorteile der Gasphasenphosgenierung sind u. a. ein verringertes Phosgenaufkommen (sog. Phosgen-"Hold-Up"), die Vermeidung schwer phosgenierbarer Zwischenprodukte, erhöhte Reaktionsausbeuten und ein geringerer Energiebedarf, da mit weniger Lösungsmittel gearbeitet wird. Die vorliegende Erfindung betrifft ausschließlich die Gasphasenphosgenierung und betrifft insbesondere ein reibungsloses Verfahren zur Inbetriebnahme einer Gasphasenphosgenierungsanlage.

Es sind aus dem Stand der Technik verschiedene Verfahren zur Herstellung von Isocyanaten durch Umsetzung von Aminen mit Phosgen in der Gasphase bekannt. Wichtig für eine gute Verfahrensführung ist eine gute Vermischung der Edukte der Gasphasenphosgenierung. EP-A-0 289 840 beschreibt die Herstellung von Diisocyanaten durch Gasphasenphosgenierung, wobei die Herstellung erfindungsgemäß in einer turbulenten Strömung bei Temperaturen zwischen 200 °C und 600 °C in einem zylindrischen Raum ohne bewegte Teile stattfindet.

EP-A-0 570 799 betrifft ein Verfahren zur Herstellung von aromatischen Diisocyanaten, bei dem die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird.

EP-A-0 699 657 beschreibt ein Verfahren zur Herstellung von aromatischen Diisocyanaten in der Gasphase, bei dem die Umsetzung des zugehörigen Diamins mit dem Phosgen in einem zwei Zonen enthaltenden Reaktor stattfindet, wobei die erste Zone, die etwa 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Bevorzugt wird die zweite Reaktionszone als Rohrreaktor ausgeführt.

Die Optimierung des Einsatzes von Rohrreaktoren für die Gasphasenphosgenierung, wie er grundlegend in der EP-A-0 570 799 unter Anwendung des Strahlmischerprinzips (Chemie-Ing.-Techn. 44 (1972) S. 1055, Abb. 10) offenbart wurde, ist Gegenstand zahlreicher Anmeldungen.

Nach der Lehre der EP-A-1 362 847 haben eine Vergleichmäßigung des über den Ringraum des Rohrreaktors zugeführten Eduktstromes und eine möglichst zentrale Zuführung beider Eduktströme in den Rohrreaktor einen großen positiven Einfluss auf die Stabilität der Reaktionszone und damit auf die Gasphasenreaktion insgesamt.

Wie in der EP-A-1 555 258 beschrieben, wird bei einer Vergrößerung der eingesetzten Rohrreaktoren auch eine Vergrößerung der Mischdüse, die häufig als Glattstrahldüse ausgebildet ist, notwendig. Mit der Vergrößerung des Durchmessers der Glattstrahldüse wird aber auch die Geschwindigkeit der Vermischung des Zentralstrahls durch den größeren erforderlichen Diffusionsweg verringert und die Gefahr von Rückvermischung erhöht, was wiederum zur Entstehung polymerer Verunreinigungen und damit fester Anbackungen im Reaktor führt. Nach der Lehre von EP-A-1 555 258 können die dargestellten Nachteile eliminiert werden, wenn der eine Eduktstrom über einen Ringspalt, der konzentrisch im Strom des anderen Eduktes angebracht ist, mit hoher Geschwindigkeit eingedüst wird. Dadurch werden der Diffusionsweg für die Vermischung klein und die Mischzeiten sehr kurz. Die Reaktion kann dann mit hoher Selektivität zum gewünschten Isocyanat ablaufen. Die Entstehung von polymeren Verunreinigungen und die Ausbildung von Anbackungen werden dadurch verringert.

Gemäß der Lehre von EP-A-1 526 129 hat eine Erhöhung der Turbulenz des Eduktstromes in der Zentraldüse einen positiven Einfluss auf die Durchmischung der Reaktanden und damit auf die Gasphasenreaktion insgesamt. Als Folge der besseren Durchmischung nimmt die Neigung zur Nebenproduktbildung ab.

EP-A-1 449 826 offenbart ein Verfahren zur Herstellung von Diisocyanaten durch Phosgenierung der entsprechenden Diamine, bei dem die dampfförmigen Diamine, gegebenenfalls verdünnt mit einem Inertgas oder mit den Dämpfen eines inerten Lösungsmittels, und Phosgen getrennt auf Temperaturen von 200 °C bis 600 °C erhitzt und in einem Rohrreaktor vermischt und zur Reaktion gebracht werden, wobei in dem Rohrreaktor eine Anzahl n ≥ 2 von parallel zur Achse des Rohrreaktors ausgerichtete Düsen angeordnet sind, wobei der die Diamine enthaltende Strom dem Rohrreaktor über die n Düsen zugeführt wird und der Phosgenstrom dem Rohrreaktor über den verbleibenden freien Raum zugeführt wird.

Eine Weiterentwicklung des Einsatzes von Rohrreaktoren für die Gasphasenphosgenierung ist Gegenstand von WO 2007/028715. Der zum Einsatz kommende Reaktor weist eine Mischeinrichtung und einen Reaktionsraum auf. Gemäß der Lehre von WO 2007/028715 umfasst der Reaktionsraum im vorderen Bereich den Mischraum, in dem überwiegend die Vermischung der gasförmigen Edukte Phosgen und Amin, gegebenenfalls vermischt mit Inertmedium, stattfindet, was in der Regel begleitet ist vom Einsetzen der Reaktion. Gemäß der Lehre von WO 2007/028715 findet im hinteren Teil des Reaktionsraumes dann im Wesentlichen nur noch die Reaktion und höchstens untergeordnet die Vermischung statt. Bevorzugt werden in dem in WO 2007/028715 offenbarten Verfahren zur Strömungsrichtung rotationssymmetrische Reaktionsräume eingesetzt, die konstruktiv im Wesentlichen in bis zu vier Längenabschnitte entlang der Längsachse des Reaktors im Verlauf der Strömung zerlegt werden können, wobei sich die Längenabschnitte in der Größe der durchströmten Querschnittsfläche unterscheiden.

WO 2008/055898 offenbart ein Verfahren zur Herstellung von Isocyanaten durch Phosgenierung der entsprechenden Amine in der Gasphase in einem Reaktor, bei dem analog zu WO 2007/028715 der zum Einsatz kommende Reaktor eine Mischeinrichtung und einen Reaktionsraum aufweist, der rotationssymmetrische Reaktionsraum konstruktiv im Wesentlichen in bis zu vier Längenabschnitte entlang der Längsachse des Reaktors im Verlauf der Strömung zerlegt werden kann, wobei sich die Längenabschnitte in der Größe der durchströmten Querschnittsfläche unterscheiden. Gegenüber WO 2007/028715 werden die Veränderungen der durchströmten Querschnittsflächen jedoch nicht durch einen in einen Rohrreaktor installierten Volumenkörper, sondern durch eine entsprechende Erweiterung bzw. Einschnürung der Reaktoraußenwandung erreicht.

EP-A-1 275 639 offenbart als mögliche Verfahrensvariante zur Herstellung von Isocyanaten durch Phosgenierung der entsprechenden Amine mit Phosgen in der Gasphase ebenfalls den Einsatz eines Reaktors, bei dem der Reaktionsraum in Strömungsrichtung hinter der Vermischung der beiden Edukte eine Erweiterung der durchströmten Querschnittsfläche aufweist. Durch eine geeignet gewählte Erweiterung der Querschnittsfläche kann die Strömungsgeschwindigkeit der Reaktionsmischung über die Länge des Reaktors gerade konstant gehalten werden. Dadurch erhöht sich die zur Verfügung stehende Reaktionszeit bei gleich bleibender Länge des Reaktors.

EP-A-2 196 455 offenbart, dass Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei die querschnittsgemittelte Strömungsgeschwindigkeit des Reaktionsgemischs längs der Achse des im Wesentlichen rotationssymmetrischen Reaktionsraumes in dem Abschnitt des Reaktionsraums, in dem der Umsatz der Aminogruppen zu den Isocyanatgruppen zwischen 4 % und 80 % liegt, maximal 8 m/sec beträgt und wobei die querschnittsgemittelte Strömungsgeschwindigkeit des Reaktionsgemisches längs der Achse des im Wesentlichen rotationssymmetrischen Reaktionsraumes in diesem Abschnitt des Reaktionsraumes stets unterhalb der querschnittsgemittelten Strömungsgeschwindigkeit am Anfang dieses Abschnittes liegt.

In EP-A-1 935 876 ist ein Gasphasenverfahren zur Herstellung von Isocyanaten durch Umsetzung entsprechender primärer Amine mit Phosgen offenbart, in dem Phosgen und die primären Amine oberhalb der Siedetemperatur der Amine innerhalb einer mittleren Kontaktzeit von 0,05 bis 15 Sekunden umgesetzt werden, wobei die Umsetzung adiabat durchgeführt wird.

In EP-A-2 408 738 ist offenbart, wie man eine Phosgendissoziation in Chlor und Kohlenmonoxid infolge einer zu langen Verweilzeit der Phosgen-haltigen Ströme bei hoher Temperatur vermeiden kann. Durch Reduzierung der Verweilzeit des Phosgens bei Temperaturen grösser als 300 °C auf maximal 5 s und durch die Limitierung der Temperatur von mit Phosgen in Kontakt stehenden Wärmeüberträgerflächen von maximal 20 K oberhalb der einzustellenden Phosgentemperatur soll dies vermieden werden.

In EP-B-1 935 875 ist ein Verfahren zur Herstellung von Isocyanaten durch Umsetzung entsprechender primärer Amine mit Phosgen in der Gasphase offenbart, bei dem zum Abbruch der Reaktion das Reaktionsgemisch aus dem Reaktionsraum heraus durch eine Kühlstrecke geführt wird, in die hinein Flüssigkeiten eingedüst werden, wobei die direkte Kühlung in der Kühlstrecke einstufig in zwei oder mehreren hintereinander geschalteten Kühlzonen erfolgt (sog. "Quenchen" des Reaktionsgemisches).

WO 2013/029918 beschreibt ein Verfahren zur Herstellung von Isocyanaten durch Umsetzung der korrespondierenden Amine mit Phosgen, das auch bei unterschiedlichen Auslastungen der Gasphasenanlage ohne Probleme durchgeführt werden kann, insbesondere soll auch bei Fahren der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Das erfindungsgemäße Verfahren soll ermöglichen, eine bestehende Anlage bei unterschiedlichen Auslastungen mit gleichbleibender Produkt- und Verfahrensqualität zu betreiben. Dies soll die Anschaffung mehrerer Anlagen mit unterschiedlichen Nennkapazitäten einsparen.

Die Anmeldung lehrt, dass wesentliche Parameter einer Phosgenierung, wie insbesondere die Verweilzeiten der Reaktionspartner in den einzelnen Apparaten, für den Betrieb der Produktionsanlage bei Nennkapazität optimiert sind, was zu Problemen hinsichtlich Ausbeute und Produktreinheit führen kann, wenn die Anlage bei geringerer als Nennkapazität betrieben wird (vgl. S. 2, Z. 20 bis 36). Um die optimierten - engen - Verweilzeitfenster auch bei Teilauslastung (d. h. gegenüber Betrieb bei Nennkapazität verringertem *Amin*strom) erreichen zu können, wird vorgeschlagen, entweder den Phosgenstrom und/oder den Inertenanteil zu erhöhen (vgl. S. 3, Z. 5 bis 19), und zwar bevorzugt so, dass der Gesamtmengenstrom aller Komponenten im Wesentlichen dem bei Nennkapazität entspricht (vgl. S. 6, Z. 4 bis 8). Die Anmeldung erwähnt zwar Abfahrprozesse in der Beschreibung des Hintergrunds der beanspruchten Erfindung auf S. 2, offenbart jedoch keinerlei technische Lehre zum konkreten Handeln, wie denn eine in Betrieb befindliche Produktionsanlage am vorteilhaftesten *außer Betrieb* genommen wird (d. h. Aminstrom und Phosgenstrom sind gleich *null*). Die in der Anmeldung offenbarten technischen Maßnahmen (d. h. die Erhöhung des Phosgenstroms und/oder des Inertenanteils) sind ausschließlich im Zusammenhang mit der Problematik des Betriebs einer Produktionsanlage bei geringerer als Nennkapazität bzw. mit der Problematik, wie eine bei Nennkapazität betriebene Anlage vorteilhafterweise auf den *Betrieb* (d. h. Amin- und Phosgenstrom sind signifikant *größer* als null) bei geringerer als Nennkapazität umgestellt werden kann (siehe die Beispiele), zu sehen.

Den beschriebenen Verfahren des Standes der Technik gelingt es zwar, eine Phosgenierung durchzuführen, ohne dass es bei den Endprodukten zu einer Qualitätseinbuße kommt, jedoch werden von wenigen Ausnahmen abgesehen nur solche Verfahren beschrieben, die sich im Normalbetrieb befinden. Der Abfahrprozess, also die sichere Außerbetriebnahme einer Gasphasenphosgenierungsanlage auf eine Art und Weise, welche eine reibungslose Wiederinbetriebnahme zu einem späteren Zeitpunkt ermöglicht, wird nicht beschrieben.

Dem Fachmann ist bekannt, dass ein kontinuierlich betriebener industrieller Prozess nicht ohne Not schlagartig außer Betrieb genommen werden sollte. Die Außerbetriebnahme (auch als Abfahren bezeichnet) einer Gasphasenphosgenierungsanlage ist ein im industriellen Alltag häufig auftretender Vorgang, der nicht zwangsläufig mit einem Öffnen oder einem anderen mechanischen Eingriff in die Phosgenieranlage verbunden sein muss. Das Abfahren zeichnet sich in der Praxis dadurch aus, dass es zu Abweichungen im Überschuss von Phosgen gegenüber Amin im Vergleich zum kontinuierlichen Betrieb bei der angestrebten Nennkapazität der Produktionsanlage kommen kann. Solche Abweichungen treten vor allen Dingen dann auf, wenn es z. B. durch Druckschwankungen zur Rückvermischung kommt. Dies ist insbesondere dann zu beobachten, wenn der aktuelle Mengenstrom an umzusetzendem Amin sehr klein ist im Vergleich zum angestrebten Mengenstrom an umzusetzendem Amin bei der angestrebten Nennkapazität der Anlage. Diese Mengenschwankungen im Verhältnis von Phosgen zu Amin sind nachteilig, da Feststoffe wie Polyharnstoff oder Aminhydrochloride ausfallen können. Darüber hinaus kann es bei nicht sachgemäßer Außerbetriebnahme zu unerwünschter Bildung von Tröpfchen an Amin kommen. Die Außerbetriebnahme einer Gasphasenphosgenierungsanlage ist daher ein kritischer Verfahrensschritt, da Fehler hierbei die spätere Wiederinbetriebnahme und die eigentliche kontinuierliche Produktion empfindlich stören können (bspw. durch Erhöhung des Differenzdrucks, welcher nötig ist, um eine ausreichende Strömung der Edukte und Produkte durch die Anlage zu gewährleisten).

Es bestand daher trotz der vielfältigen Fortschritte auf dem Gebiet der Gasphasenphosgenierung Bedarf an weiteren Verbesserungen. Diesem Bedarf Rechnung tragend ist ein Gegenstand der vorliegenden Erfindung ein Verfahren zum Betreiben einer Gasphasenphosgenierungsanlage (**100**) zur Umsetzung eines Amins (**2**) mit Phosgen (**1**) im stöchiometrischen Überschuss in Bezug auf die primären Aminogruppen des Amins (**2**) zum korrespondierenden Isocyanat (**4**), wobei die Gasphasenphosgenierungsanlage (**100**) mindestens
(i) eine Vorrichtung **1000** zur Bereitstellung eines gasförmigen Phosgenstroms (**10**), optional umfassend neben Phosgen (**1**) einen Inertstoff (**3**),
(ii) eine Vorrichtung **2000** zur Bereitstellung eines gasförmigen Aminstroms (**20**), optional umfassend neben Amin (**2**) einen Inertstoff (**3**),
(iii) eine Mischzone (**3100**) zur Vermischung der Ströme **10** und **20,** wobei die Mischzone durch Einrichtungen (**1100**, **2100**) jeweils mit der Vorrichtung **1000** und der Vorrichtung **2000** verbunden ist,
(iv) eine strömungstechnisch nach der Mischzone (**3100**) angeordnete Reaktionszone (**3200**) zur weiteren Umsetzung der zuvor vermischten Ströme **10** und **20,**
(v) eine strömungstechnisch nach der Reaktionszone (**3200**) angeordnete Reaktionsabbruchzone (**4000**) zur Beendigung der Umsetzung, und optional
(vi) einen Aufarbeitungsteil (**5000**) umfassend Einrichtungen zur Rückgewinnung und Rückführung nicht umgesetzten Phosgens (**1"**) (**5100**) und Einrichtungen zur Gewinnung des hergestellten Isocyanats in Reinform (**5200**)
umfasst,
bei dem im Regelbetrieb der Gasphasenphosgenierungsanlage (**100**) das Phosgen (**1**) im Phosgengasstrom (**10**) bevorzugt eine Mischung aus Frisch-Phosgen (**1'**) und den Einrichtungen (**5100**) zurückgewonnenem Rezyklat-Phosgen (**1**") ist,
wobei
zur Außerbetriebnahme der Gasphasenphosgenierungsanlage (**100**) die folgenden Schritte durchlaufen werden:
(I) Reduzierung des Amin-Massenflusses M'(**2**) auf null, wobei ein gasförmiger Inertgasstrom (**30**) durch die Einrichtung **2100,** die Mischzone (**3100**), die Reaktionszone (**3200**) und die Reaktionsabbruchzone (**4000**) aufrechterhalten wird;
(II) erst nach Verstreichen eines Zeitraums, der mindestens der 10-fachen Verweilzeit, bevorzugt mindestens der 30-fachen Verweilzeit, besonders bevorzugt mindestens der 60-fachen Verweilzeit, ganz besonders bevorzugt mindestens der 90-fachen Verweilzeit, von Phosgen (**1**) von Austritt aus der Vorrichtung **1000** bis Austritt aus der Reaktionsabbruchzone (**4000**) im Regelbetrieb der Gasphasenphosgenierungsanlage (**100**) entspricht, gerechnet ab dem Zeitpunkt, ab dem M'(**2**) null beträgt, Reduzierung des Massenflusses an Phosgen M'(**1**), welcher die Vorrichtung **1000** verlässt, auf null, wobei ein gasförmiger Inertgasstrom (**30**) durch die Einrichtung **1100,** die Mischzone (**3100**), die Reaktionszone (**3200**) und die Reaktionsabbruchzone (**4000**) aufrechterhalten wird;
(III) Aufrechterhalten zumindest des Inertgasstroms (**30**) aus (I), bevorzugt der Inertgasströme (**30**) aus (I) und (II), mindestens für einen Zeitraum, welcher der dreifachen, bevorzugt mindestens der 100-fachen, besonders bevorzugt mindestens der 200-fachen, ganz besonders bevorzugt mindestens der 350-fachen, Verweilzeit des Inertgasstroms (**30**) von Eintritt in die Mischzone (**3100**) bis Austritt aus der Reaktionsabbruchzone (**4000**) entspricht, gerechnet ab dem Zeitpunkt, ab dem der Massenfluss an Phosgen M'(**1**), welcher die Vorrichtung **1000** verlässt, null beträgt.

Unter einer *Gasphasenphosgenierung* ist erfindungsgemäß eine Verfahrensführung zur Phosgenierung von Aminen zu den korrespondierenden Isocyanaten zu verstehen, bei der die Amine im gasförmigen Zustand zu den Isocyanaten reagieren und im Verlauf der Reaktion sämtliche vorhandenen Komponenten (Edukte, Produkte, Zwischenprodukte, ggf. Nebenprodukte, ggf. Inertstoffe) während des Durchgangs durch die Reaktionszone zu mindestens 95,0 Massen-%, bevorzugt zu mindestens 98,0 Massen-%, besonders bevorzugt zu mindestens 99,0 Massen-%, ganz besonders bevorzugt zu mindestens 99,8 Massen-% und speziell zu mindestens 99,9 Massen-% jeweils bezogen auf die Gesamtmasse aller vorhandenen Komponenten, in der Gasphase verbleiben.

Die *Verweilzeit* einer Komponente in einem Anlagenteil bezeichnet den Quotienten aus Volumenstrom der Komponente im Normzustand (angegeben als *Vₙ*/h, wobei *Vₙ* für m3 bei Normbedingungen steht ("Normkubikmeter")) und dem durchströmten Innenvolumen des betreffenden Anlagenteils (angegeben in m³). Ein Normkubikmeter *Vₙ* ist dabei diejenige Gasmenge, die bei
- einem Druck *pₙ* von 1,01325 bar,
- einer Luftfeuchtigkeit von 0 % (trockenes Gas) und
- einer Temperatur *Tₙ* von 273,15 K (*tₙ* = 0 °C) (Normbedingungen nach DIN 1343, STPD)
ein Volumen *V* von einem Kubikmeter hätte.

Die in Schritt (II) relevante Verweilzeit des Phosgens (**1**) im Regelbetrieb der Gasphasenphosgenierungsanlage (**100**) ist eine rechnerische Größe, die aus dem bekannten Volumenstrom des Phosgens, das im Regelbetrieb die Vorrichtung **1000** verlässt (ohne evtl. vorhandenen Inertstoffanteil), und dem bekannten Innenvolumen der durchströmten Apparate inklusive der diese verbindenden Rohrleitungen ermittelt wird. In analoger Weise wird die in Schritt (III) relevante Verweilzeit des Inertgasstroms (**30**) rechnerisch ermittelt. Dabei wird der Volumenstrom an Inertgasstrom (**30**) zugrunde gelegt, welcher zu dem Zeitpunkt, an dem der Massenstrom M'(**1**), welcher die Vorrichtung **1000** verlässt, null erreicht, vorliegt. Dieser Inertgasstrom (**30**) besteht in der einfachsten Ausführungsform aus dem Inertgasstrom aus (I). Bevorzugt wird jedoch in (III) auch der Inertgasstrom aus (II) noch aufrechterhalten. In dieser letztgenannten Ausführungsform besteht der Inertgasstrom in (III) daher aus den vereinigten Inertgasströmen aus (I) und (II).

Geeignete *Amine (**2**)* sind dabei insbesondere Isophorondiamin, Hexamethylendiamin, Bis(p-aminocyclohexyl)methan, Toluylendiamin und Diphenylmethandiamin.

Im Rahmen der vorliegenden Erfindung umfasst der Ausdruck *"Außerbetriebnahme einer Gasphasenphosgenierungsanlage"* alle Verfahrensschritte, die erforderlich sind, um eine in Betrieb befindliche Gasphasenphosgenierungsanlage (die mit einer zu einem bestimmten Zeitpunkt angestrebten Sollproduktion, ausgedrückt als angestrebter Massenfluss an umzusetzendem Amin, M'_{Soll}(**2**) [z. B. t(Amin)/h], betrieben wird), außer Betrieb zu nehmen, beispielsweise um Wartungsarbeiten durchzuführen. Der Betrieb der Gasphasenproduktionsanlage (**100**) bei M'_{Soll}(**2**) wird im Rahmen dieser Erfindung als *Regelbetrieb* bezeichnet. M'_{Soll}(**2**) kann, muss aber nicht, dabei dem Wert von M'_{Soll}(**2**) bei Nennkapazität M'_{Nenn}(**2**) der Gasphasenproduktionsanlage (**100**) entsprechen. Die Nennkapazität einer Produktionsanlage wird in der Fachwelt in pro Jahr zu produzierender Tonnen an Produkt ("Jahrestonnen") angegeben, wobei alle planbaren Anlagenstillstände berücksichtigt werden.

Das Wort *"ein"* ist im Rahmen dieser Erfindung im Zusammenhang mit zählbaren Größen lediglich als unbestimmter Artikel und nur dann als Zahlwort zu verstehen, wenn dies ausdrücklich, etwa durch den Zusatz *"genau* ein", gesagt wird. Bspw. schließt der Ausdruck *"eine Reaktionszone"* die Möglichkeit des Vorhandenseins mehrerer (in Reihe oder parallel geschalteter) Reaktionszonen nicht aus.

Erfindungsgemäß wesentlich ist, dass zur Außerbetriebnahme (zum "Abfahren") der Gasphasenphosgenierungsanlage (**100**) zunächst nur der Amin-Massenfluss M'(**2**) vom Wert M'_{Soll}(**2**) auf null reduziert wird. Beim Abfahren wird das Amin immer vor dem Phosgen abgestellt, wodurch ein Rückvermischen von Amin in die Phosgenzuführungseinrichtungen verhindert und ein Überschuss an Phosgen gegenüber Amin auch während der Außerbetriebnahme der Gasphasenphosgenierungsanlage (**100**) sichergestellt wird. Durch das erfindungsgemäße Durchspülen der Aminzuführungseinrichtungen mit einem Inertgasstrom (**30**) wird ein Zurückströmen von Phosgen in die Aminzuführungseinrichtungen (Rückvermischung) verhindert. Das Durchspülen der Einrichtung **2100** (und bevorzugt auch der Einrichtung **1100**), der Mischzone (**3100**), der Reaktionszone (**3200**) und der Reaktionsabbruchzone (**4000**) mit einem Inertgasstrom (**30**) wird frühestens nach einem Zeitraum eingestellt, welcher der dreifachen, bevorzugt der 100-fachen, besonders bevorzugt der 200-fachen, ganz besonders bevorzugt der 350-fachen Verweilzeit des Inertgasstroms (**30**) aus (I), in der bevorzugten Ausführungsform der vereinigten Inertgasströme (**30**) aus (I) und (II), von Beginn der Mischzone (**3100**) bis Austritt aus der Reaktionsabbruchzone (**4000**) entspricht, gerechnet ab dem Zeitpunkt, ab dem der Massenfluss an Phosgen M'(**1**), welcher die Vorrichtung **1000** verlässt, null beträgt.

Nachstehend werden die Schritte der Erfindung detailliert erläutert. Verschiedene Ausführungsformen sind dabei beliebig miteinander kombinierbar, solange sich für den Fachmann aus dem Kontext nicht eindeutig das Gegenteil ergibt.

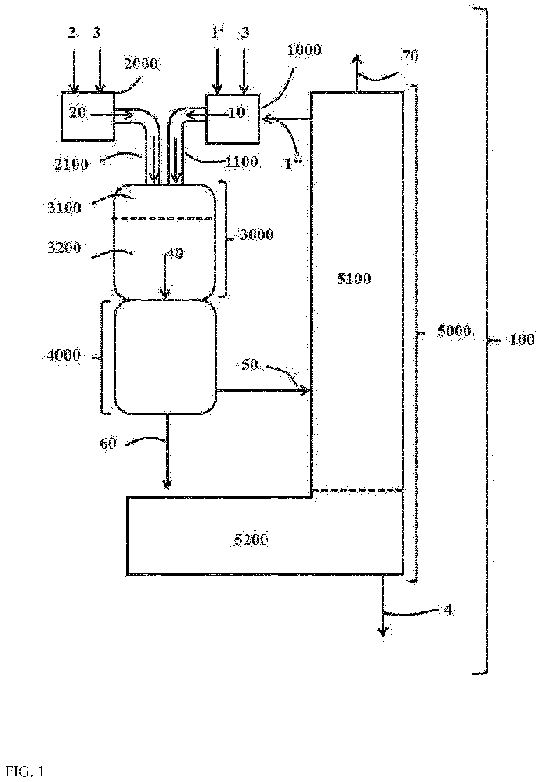

Erfindungsgemäß umfasst eine Gasphasenphosgenierungsanlage (**100**) mindestens die oben unter **(i) bis (v) aufgeführten Einrichtungen** (vgl. auch FIG. 1, welche die Einrichtungen einer erfindungsgemäß einzusetzenden Gasphasenphosgenierungsanlage (**100**) inklusive des bevorzugt vorhandenen Aufarbeitungsteils sowie die Stoffströme im Regelbetrieb zeigt).
(i) Als *Vorrichtung zur Bereitstellung eines gasförmigen Phosgenstroms (1000)* kann grundsätzlich jede aus dem Stand der Technik bekannte, für die Überführung von Phosgen in die Gasphase geeignete, Vorrichtung verwendet werden. Bevorzugt erfolgt die Phosgengaserzeugung durch Destillation oder partielle Verdampfung in einer Destillationskolonne, wie in DE102009032413A1 in den Abschnitten [0081] bis [0118] beschrieben. Die Energiezufuhr im Sumpf der Kolonne kann durch jeden denkbaren Verdampfer erfolgen, wie zum Beispiel Naturumlaufverdampfer, Kletterverdampfer und Fallfilmverdampfer. Insbesondere bevorzugt sind Fallfilmverdampfer.
(ii) Als *Vorrichtung zur Bereitstellung eines gasförmigen Aminstroms **(2000)*** kann grundsätzlich jede aus dem Stand der Technik bekannte, für die Überführung eines Amins in die Gasphase geeignete, Vorrichtung, wie dem Fachmann bekannte Verdampfungsapparaturen, verwendet werden. In einer bevorzugten Ausführungsform umfasst die Vorrichtung **2000** eine Einrichtung zur Verdampfung und eine Einrichtung zur anschließenden Überhitzung des Amins (**2**). Ganz besonders bevorzugt sind mehrstufige Verdampfungs- und Überhitzungssysteme, in denen zwischen den Verdampfungs- und Überhitzungssystemen Tröpfchenabscheider eingebaut sind und/oder die Verdampfungsapparaturen auch die Funktion eines Tröpfchenabscheiders besitzen. Geeignete Tröpfchenabscheider sind z. B. beschrieben in "Droplet Separation", A. Bürkholz, VCH Verlagsgesellschaft, Weinheim - New York - Basel - Cambridge, 1989. Nach Verlassen des in Strömungsrichtung letzten Überhitzers wird der auf seine Solltemperatur vorgeheizte gasförmige Eduktstrom (**20**) dem Reaktionsraum zugeführt.
(iii) Der Aufbau einer erfindungsgemäß einsetzbaren *Mischzone (**3100**)* kann auf dem Fachmann bekannte Art und Weise erfolgen, bevorzugt wie in EP-A-2 196 455, insbesondere in den Absätzen [0047] bis [0049], und EP-A-1 935 876, insbesondere in den Absätzen [0027] bis [0029], beschrieben.

Die Mischzone beginnt dort, wo im Regelbetrieb die Ströme (**10**) und (**20**) erstmals aufeinandertreffen. An dieser Stelle, auch als Eintritt in die Mischzone (**3100**) bezeichnet, vermischen sich während des Vorgangs der Außerbetriebnahme nach Einleitung von Schritt (II) die Inertgasströme aus (I) und (II) zu einem gemeinsamen Inertgasstrom, dessen Verweilzeit bis zum Austritt aus der Reaktionsabbruchzone (**4000**) die Grundlage für die Berechnung der Mindestdauer von Schritt (III) bildet.
(iv) Die in der Mischzone (**3100**) erstmals aufeinandertreffenden Amin- und Phosgengasströme werden in einem Verweilzeitapparat, der *Reaktionszone (**3200**),* weiter umgesetzt. Mischzone (**3100**) und Reaktionszone (**3200**) können bevorzugt auch in einem einzigen Apparat, dem *Reaktor (**3000**)* vereinigt sein wie in EP 2 196 455 A1, insbesondere in den Absätzen [0042] bis [0049], beschrieben.

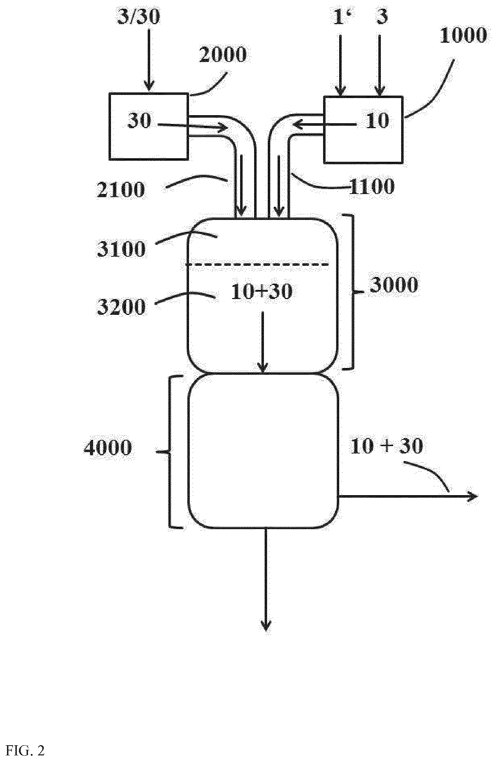

Die *Einrichtungen **1100** und **2100***, welche die Vorrichtungen zur Bereitstellung des gasförmigen Phosgen- (**1000**) bzw. Amingasstroms (**2000**) mit der Mischzone (**3100**) verbinden, sind erfindungsgemäß solche Einrichtungen, welche sich zur Überführung des jeweiligen Gasstroms (**10**) bzw. (**20**) aus den Vorrichtungen **1000** bzw. **2000** in die Mischzone (**3100**) eignen. Diese Einrichtungen umfassen neben Rohrleitungen zum Transport der Gasströme bevorzugt auch Düsenvorrichtungen, die eine intensive Durchmischung von Phosgengasstrom (**10**) und Amingasstrom (**20**) in der Mischzone (**3100**) gewährleisten. Es ist möglich, jeden der Gasströme (**10**) und (**20**) einzeln in die Mischzone (**3100**) einzudüsen. Bevorzugt ist jedoch eine Ausführungsform, in welcher die Rohrleitungen der Einrichtungen **1100** und **2100** in eine gemeinsame Düsenvorrichtung einmünden (in FIG. 1 nicht gezeigt). In dieser Ausführungsform wird einer der beiden Gasströme, bevorzugt der Amingasstrom (**20**), der Mischzone (**3100**) über eine zentral in einem bevorzugt zylindrischen Behälter angeordnete Innendüse zugeführt. Der andere Gasstrom, bevorzugt der Phosgengasstrom (**10**), wird über den durch die Außenwand der Innendüse und der Innenwand des Behälters gebildeten Ringraum eingetragen. An der Austrittsöffnung der Innendüse (= Beginn der Mischzone) vermischen sich die beiden Gasströme. Eine solche Ausführungsform ist beispielsweise in FIG. 1 von EP-A-1 449 826 dargestellt. In diesem Fall sind die Einrichtungen **1100** und **2100** teilweise in einander und in die Mischzone (**3100**) integriert. Es ist auch möglich, wie in FIG. 2 von EP-A-1 449 826 dargestellt, an Stelle einer einzigen Zentraldüse eine Anordnung aus mehreren Einzeldüsen zu verwenden. Weitere erfindungsgemäß einsetzbare Ausführungsformen für die Einrichtungen **1100** und **2100** sind beispielsweise in EP-A-2 196 455, insbesondere in den Absätzen [0047] bis [0048], und EP-A-1 935 876, insbesondere in den Absätzen [0027] und [0028], beschrieben.
(v) Erfindungsgemäß einsetzbare *Reaktionsabbruchzonen **(4000)*** sind dem Fachmann bekannt. Bevorzugt ist eine Ausführungsform, wie sie in EP 1 935 875 B1, insbesondere in den Absätzen [0024] und [0025], beschrieben ist. In der Reaktionsabbruchzone (**4000**) wird das Rohprodukt der Reaktion (**40**), welches neben dem Isocyanat (**4**) im Wesentlichen noch das Koppelprodukt Chlorwasserstoff und nicht umgesetztes Phosgen enthält, rasch abgekühlt, bevorzugt durch Eindüsen eines inerten Lösungsmittels (bevorzugt *ortho*-Dichlorbenzol, ODB), ggf. gemeinsam mit einem Teil zuvor gebildeten und rezyklierten Isocyanats (**4**), in den Gasstrom (**40**). Bevorzugt wird das rohe Reaktionsprodukt (**40**) in der Reaktionsabbruchzone (**4000**) in einen gasförmigen Anteil (Brüden, **50**) und einen flüssigen Anteil (**60**) getrennt. Bevorzugt wird die Reaktionsabbruchzone frühestens dann außer Betrieb genommen, wenn der Massenfluss an Phosgen M'(**1**), welcher die Vorrichtung **1000** verlässt, null beträgt. Besonders bevorzugt wird die Reaktionsabbruchzone erst nach einem Zeitraum, welcher mindestens der dreifachen, bevorzugt mindestens der 100-fachen, besonders bevorzugt mindestens der 200-fachen, ganz besonders bevorzugt mindestens der 350-fachen Verweilzeit des Inertgasstroms (**30**) von Eintritt in die Mischzone (**3100**) bis Austritt aus der Reaktionsabbruchzone (**4000**) entspricht, gerechnet ab dem Zeitpunkt, ab dem der Massenfluss an Phosgen M'(**1**), welcher die Vorrichtung **1000** verlässt, null beträgt.

In einer besonders bevorzugten Ausgestaltung des erfindungsgemäßen Verfahrens wird das in der Reaktionsabbruchzone (**4000**) erhaltene Rohprodukt in derselben Gasphasenphosgenierungsanlage (**100**) aufgearbeitet, um das Isocyanat (**4**) aus dem flüssigen Gemisch (**60**) zu isolieren. In diesem Fall umfasst die Gasphasenphosgenierungsanlage (**100**) zusätzlich
(vi) einen *Aufarbeitungsteil **(5000)**.*

Geeignete Vorrichtungen zur Aufarbeitung sind in WO 2011/003532, insbesondere Seite 5, Zeile 19 bis Seite 28, Zeile 5, und in EP 1 371 636 B1, EP 1 371 635 B1 und EP 1 413 571 B1, jeweils das gesamte Dokument, beschrieben. Der Aufarbeitungsteil (**5000**) lässt sich unterteilen in *Einrichtungen zur Rückgewinnung und Rückführung nicht umgesetzten Phosgens (sowie zur Abtrennung des Koppelprodukts Chlorwasserstoff) (**5100**)* und in *Einrichtungen zur Gewinnung des hergestellten Isocyanats in Reinform (sowie ggf. zur Rückgewinnung inerten Lösungsmittels) (**5200**).* Der Aufarbeitungsteil ist in FIG. 1 nur schematisch angedeutet ohne die nachfolgend aufgeführten Details. Insbesondere bevorzugt umfasst der Aufarbeitungsteil (**5000**) in den Einrichtungen **5100** eine *Auswaschkolonne (**5110**)* zur Entfernung von Isocyanat aus den Brüden (**50**) der Reaktionsabbruchsszone (**4000**) durch Waschen mit einem inertem Lösungsmittel, eine *Phosgenabsorptionskolonne (**5120**)* zur Wiedergewinnung von Phosgen aus den Brüden der Auswaschkolonne (**5110**) durch Absorption in einem inerten Lösungsmittel, wodurch Chlorwasserstoff und Inerte (**70**) vom Phosgen getrennt werden, eine *Phosgendesorptionskolonne (**5130**)* zur Trennung von Phosgen und inertem Lösungsmittel, sowie in den Einrichtungen **5200** eine *Lösungsmittelkolonne (**5210**)* insbesondere zur Abtrennung von Leichtsiedern (insbesondere inertem Lösungsmittel aus der Reaktionsabbruchzone) aus dem rohen Isocyanat, eine *Feinreinigungskolonne (**5220**)* insbesondere zur Abtrennung von Schwersiedern (z. B. Polyharnstoff-haltigen Rückständen) aus dem in der Lösungsmittelkolonne vorgereinigten Isocyanat, so dass gereinigtes Endprodukt erhalten wird.

Es ist möglich (in FIG. 1 nicht gezeigt), die Vorrichtung (**1000**) zur Bereitstellung eines gasförmigen Phosgenstroms (**10**) derart in die Phosgendesorptionskolonne (**5130**) zu integrieren, dass der aus der Aufarbeitung stammende lösungsmittelhaltige Phosgenstrom gemeinsam mit Frisch-Phosgen (**1'**) verdampft und in der Phosgendesorptionskolonne (**5130**) destilliert wird. Das erhaltene gasförmige Phosgen wird über die Einrichtung **1100** der Mischzone zugeführt, während das abgetrennte inerte Lösungsmittel bevorzugt in die Auswaschkolonne (**5110**) und/oder die Phosgenabsorptionskolonne **(5120)** geführt wird.

Bei Betrieb der Gasphasenphosgenierungsanlage (**100**) mit dem angestrebten Soll-Massenfluss an umzusetzendem Amin von M'_{Soll}**(2)** werden die Zusammensetzungen und Massenflüsse der Ströme **10** und **20** bevorzugt so aufeinander abgestimmt, dass in Strom **10** Phosgen (**1**) in einem stöchiometrischen Überschuss in Bezug auf die primären Aminogruppen des Amins (**2**) in Strom **20** von mindestens 150 % der Theorie, besonders bevorzugt 160 % bis 350 % der Theorie und ganz besonders bevorzugt 170 % bis 300 % der Theorie, vorliegt. Theoretisch reagiert ein Mol Phosgen mit einem Mol Aminogruppen, d. h., theoretisch reagieren zwei Mol Phosgen mit einem Mol eines Diamins. Der angestrebte Soll-Massenfluss M'ₛₒₗₗ**(2)** kann dabei der Nennkapazität der Gasphasenphosgenierungsanlage unter Zugrundelegung einer üblicher Anlagenverfügbarkeit entsprechen, d. h. der in einem bestimmten Zeitraum produzierten Menge an Isocyanat, für welche die Anlage konstruiert wurde (in der Regel als Tonnen pro Jahr angegeben). Wenn ungünstige Absatzbedingungen dies erforderlich machen, kann der angestrebte Soll-Massenfluss M'_{Soll}(**2**) natürlich auch geringer sein. Dies ist für das erfindungsgemäße Verfahren nicht wesentlich. Beispielsweise ist es durchaus denkbar, das erfindungsgemäße Verfahren auf eine Gasphasenproduktionsanlage (**100**) anzuwenden, welche infolge eines schwierigen Marktumfelds zeitweise nur bei 50 % der Nennkapazität betrieben wird. Für die Dauer der schwierigen Marktsituation entspricht dann der Regelbetrieb der Produktion einem Amin-Massenfluss M'_{Soll}(**2**) = 0,5 M'_{Nenn}(**2**)**.**

Der gasförmige Phosgenstrom (**10**) kann neben Phosgen (**1**) noch einen Inertstoff (**3**) enthalten. Erfindungsgemäß einsetzbare Inertstoffe (**3**) sind dabei neben solchen Stoffen, die bereits bei Raumtemperatur und Normaldruck gasförmig sind, wie Stickstoff, Helium oder Argon, auch die Dämpfe inerter organischer Lösungsmittel, die bei Raumtemperatur und Normaldruck flüssig sind, z. B. aromatische Kohlenwasserstoffe, ggf. mit Halogensubstitution, wie z. B. Chlorbenzol oder Dichlorbenzol (alle Isomere, bevorzugt *ortho*-Dichlorbenzol). Besonders bevorzugt wird Stickstoff zur Verdünnung des Phosgens eingesetzt. Der Anteil an Inertstoff (**3**) im Phosgengasstrom (**10**) kann dabei wie im Stand der Technik üblich gewählt werden.

Ebenso kann der gasförmige Aminstrom (**20**) neben Amin (**2**) noch einen Inertstoff (**3**) enthalten. Hierbei sind die gleichen Inertstoffe (**3**), wie zuvor für den Phosgengasstrom (**10**) beschrieben, möglich. Enthalten beide Ströme (**10** und **20**) einen Inertstoff (**3**), so wird bevorzugt der gleiche Inertstoff (**3**) eingesetzt.

Der Betrieb der Gasphasenphosgenierungsanlage (**100**) bei einem angestrebten Wert für den Soll-Massenfluss M'_{Soll}(**2**) kann nach einem aus dem Stand der Technik bekannten Verfahren erfolgen. Bevorzugt wird hierzu das in der Mischzone (**3100**) erzeugte Reaktionsgemisch unter Vermeidung von Rückvermischung kontinuierlich durch die Reaktionszone (**3200**) geleitet und darin bevorzugt bei einer Temperatur von 200 °C bis 600 °C und einem absoluten Druck von 80 mbar bis 2.500 mbar innerhalb einer mittleren Kontaktzeit von 0,05 bis 15 Sekunden adiabat oder isotherm, bevorzugt adiabat, zu einem gasförmigen Verfahrensprodukt, enthaltend das gewünschte Isocyanat (**4**), umgesetzt. Geeignete Ausführungsformen sind in EP 2 196 455 B1 und EP 1 935 876 B1 beschrieben.

In der Reaktionsabbruchzone (**4000**) wird das aus der Reaktionszone (**3200**) austretende, gasförmige Verfahrensprodukt (**40**), rasch abgekühlt. Bevorzugt geschieht dies durch Kontaktieren mit einem inerten Lösungsmittel, dessen Temperatur unterhalb der Siedetemperatur des Isocyanats (**4**) und oberhalb der Zersetzungstemperatur des dem umgesetzten Amin entsprechenden Carbaminsäurechlorids gehalten wird. Geeignete Ausführungsformen sind in EP 1 935 875 B1 beschrieben. In diesem Schritt ggf. nicht kondensiertes Isocyanat (**4**) wird bevorzugt durch Waschen mit einer Waschflüssigkeit aus dem in der Reaktionsabbruchzone verbliebenem Gasgemisch abgetrennt und bevorzugt mit dem in der Reaktionsabbruchzone (**4000**) erhaltenem Kondensat (**60**) vereinigt. Eine geeignete Ausführungsform ist in EP 1 935 875 B1, insbesondere in den Absätzen [0024] und [0025], beschrieben.

Im Anschluss wird aus dem so erhaltenen rohen flüssigen Verfahrensprodukt (**60**) das gewünschte Isocyanat (**4**) durch Destillation isoliert. Geeignete Ausführungsformen sind dem Fachmann bekannt und beispielsweise in WO 2013/139703, EP 1 413 571 B1, EP 1 371 635 B1, EP 1 371 636 B1 beschrieben.

Die Hauptmenge des in der Reaktion nicht umgesetzten Phosgens verbleibt in der Gasphase (**50**), welche in der Reaktionsabbruchzone (**4000**) erhalten wird. Es ist bevorzugt, diese Gasphase in den Einrichtungen **5100** des bevorzugt vorhandenen Aufarbeitungsteils **5000** aufzuarbeiten, um dieses Phosgen rezyklieren zu können. Dieser Schritt ist aus dem Stand der Technik hinlänglich bekannt. Geeignete Ausführungsformen wurden weiter oben bereits beschrieben.

Zur Außerbetriebnahme einer so betriebenen Gasphasenphosgenierungsanlage werden nun erfindungsgemäß die folgenden Schritte durchlaufen:
In **Schritt (I)** des erfindungsgemäßen Verfahrens erfolgt die Abschaltung der Aminzufuhr in die Misch- und Reaktionszone. Dies geschieht durch eine Reduzierung des in die Vorrichtung **2000** eingetragenen Aminmassenstroms M'(**2**) vom Wert M'_{Soll}(**2**) auf null. Diese Reduzierung erfolgt stufenlos oder in Stufen, bevorzugt stufenlos.

Während dieses Schrittes (I) wird ein Inertgasstrom (**30**) durch die Einrichtung **2100,** die Mischzone (**3100**), die Reaktionszone (**3200**) und die Reaktionsabbruchzone (**4000**) aufrechterhalten (vgl. auch FIG. 2). Der Inertgasstrom (**30**) besteht aus mindestens einem Inertstoff (**3**), wobei die gleichen Inertstoffe geeignet sind, wie sie weiter oben für die optional erfolgende Verdünnung des Phosgens im Gasstrom (**10**) beschrieben wurden. Bevorzugt hat dieser Inertgasstrom (**30**) eine Temperatur oberhalb des Taupunktes des Amins (**2**) unter den vorliegenden Druckverhältnissen. Bevorzugt ist die Temperatur aber nicht so hoch, dass eine thermische Zersetzung der noch in den Apparaten befindlichen Edukte bzw. Produkte zu befürchten wäre. Erfindungsgemäß bevorzugt ist eine Temperatur im Bereich von 200 °C bis 600 °C, besonders bevorzugt im Bereich von 200 °C bis 500 °C und ganz besonders bevorzugt im Bereich von 250 °C bis 500 °C.

Die Aufrechterhaltung des Inertgasstroms (**30**) in Schritt (I) kann geschehen, indem (**a**) ein bei Raumtemperatur und Normaldruck bevorzugt flüssig vorliegender Inertstoff (**3**) einer Temperatur T₃, welche kleiner als die für den Inertgasstrom (**30**) in Schritt (I) angestrebte Temperatur (d. h. bevorzugt kleiner als 200 °C) ist, in die Vorrichtung **2000** eingeleitet, dort erhitzt und anschließend in die Einrichtung **2100** und die weiteren zu inertisierenden Anlagenteile geleitet wird. In der Variante (a) wird also der Inertgasstrom (**30**) in der Vorrichtung **2000** bereitgestellt. Diese Ausführungsform ist insbesondere dann vorteilhaft, wenn das Reaktionsgemisch während der Reaktion im Regelbetrieb mit den Dämpfen eines bei Raumtemperatur und Normaldruck flüssig vorliegenden Inertstoffs (**3**) verdünnt wird. Es ist möglich, den Inertstoff (**3**) flüssig in die Vorrichtung **2000** einzutragen und dort erst zu verdampfen. In der Variante (a) besonders geeignete Inertstoffe (**3**) sind inerte Lösungsmittel wie aromatische Kohlenwasserstoffe, ggf. mit Halogensubstitution, wie z. B. Chlorbenzol oder Dichlorbenzol (alle Isomere, bevorzugt *ortho*-Dichlorbenzol). In der Vorrichtung **2000** wird der Inertstoff (**3**) so erhitzt (d. h. bei Eintrag als Flüssigkeit verdampft), dass ein Inertgasstrom (**30**) erhalten wird, der eine Temperatur T₃₀ von bevorzugt 200 °C bis 600 °C, besonders bevorzugt 200 °C bis 500 °C und ganz besonders bevorzugt 250 °C bis 500 °C, und einen absoluten Druck p₃₀ von bevorzugt 100 mbar bis 3.000 mbar, besonders bevorzugt 150 mbar bis 2.800 mbar und ganz besonders bevorzugt 200 mbar bis 2.500 mbar, aufweist. Die Ströme (**3**) und (**30**) unterscheiden sich nicht in ihrer chemischen Zusammensetzung, sondern lediglich in Temperatur und ggf. Druck. Nachdem der in die Vorrichtung **2000** eingetragene Strom (**3**) erhitzt wurde, so dass er die Vorrichtung **2000** gasförmig verlässt, wird er als Strom (**30**) bezeichnet.

Es ist **(b)** auch möglich, in die Vorrichtung **2000** direkt einen Inertgasstrom (**30**) einer Temperatur T₃₀ von bevorzugt 200 °C bis 600 °C, besonders bevorzugt 200 °C bis 500 °C und ganz besonders bevorzugt 250 °C bis 500 °C, und eines absoluten Drucks p₃₀ von bevorzugt 100 mbar bis 3.000 mbar, besonders bevorzugt 150 mbar bis 2800 mbar und ganz besonders bevorzugt 200 mbar bis 2500 mbar, einzuleiten und von dort in die Einrichtung **2100** und die anderen zu inertisierenden Anlagenteile zu überführen. Es ist in dieser Ausführungsform auch möglich, den eingeleiteten Inertgasstrom (**30**) in der Vorrichtung **2000** im Rahmen der zuvor definierten Temperaturbereiche weiter aufzuheizen. Alternativ kann der Inertgasstrom (**30**) auch direkt in die Einrichtung **2100** eingespeist werden. In dieser letztgenannte Ausführungsform wird bevorzugt nach Reduzierung des Massenflusses an Amin M'**(2)** auf null die Rohrleitung zwischen der Vorrichtung **2000** und der Mischzone **(3100),** welche Bestandteil der Einrichtung **2100** ist, geschlossen, insbesondere bevorzugt unmittelbar hinter der Vorrichtung **2000.** Variante (a), egal in welcher Ausgestaltung, ist insbesondere dann vorteilhaft, wenn das Reaktionsgemisch während der Reaktion im Regelbetrieb mit einem bei Raumtemperatur und Normaldruck bereits gasförmig vorliegenden Inertstoff wie Stickstoff, Helium oder Argon verdünnt wird.

Bis zur vollständigen Unterbrechung der Zufuhr von Amin (**2**) aus der Vorrichtung **2000** wird bevorzugt der Druck in der Vorrichtung **2000** p₂₀₀₀ durch Einspeisung des Inertstoffstroms (**30**) auf einen Wert eingestellt, der oberhalb des Drucks p₃₁₀₀ in der Mischzone **(3100)** liegt. Dabei beträgt der Wert für p₃₁₀₀ bevorzugt 80 mbar (absolut) bis 2.500 mbar (absolut), während der Wert p₂₀₀₀ bevorzugt 100 mbar (absolut) bis 3.000 mbar (absolut) beträgt.

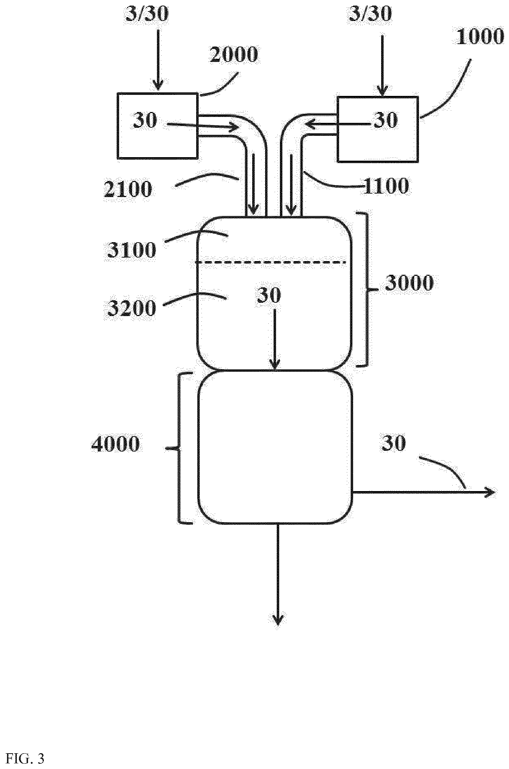

In **Schritt (II)** des erfindungsgemäßen Verfahrens wird der Massenfluss an Phosgen M'(**1**), welcher die Vorrichtung **1000** verlässt, vom Wert M'_{Soll}**(1)** auf null reduziert. Dies geschieht stufenlos oder in Stufen, bevorzugt stufenlos. Schritt (II) wird erst nach Verstreichen eines Zeitraums, der mindestens der 10-fachen Verweilzeit, bevorzugt mindestens der 30-fachen Verweilzeit, besonders bevorzug mindestens der 60-fachen Verweilzeit, ganz besonders bevorzugt mindestens der 90-fachen Verweilzeit, von Phosgen (**1**) von Austritt aus der Vorrichtung **1000** bis Austritt aus der Reaktionsabbruchzone (**4000**) im Regelbetrieb der Gasphasenphosgenierungsanlage (**100**) entspricht, gerechnet ab dem Zeitpunkt, ab dem M'**(2)** null beträgt, durchgeführt. Dabei wird ein gasförmiger Inertgasstrom (**30**) durch die Einrichtung **1100,** die Mischzone (**3100**), die Reaktionszone (**3200**) und die Reaktionsabbruchzone (**4000**) aufrechterhalten (vgl. auch FIG. 3). Der Inertgasstrom (**30**) besteht aus mindestens einem gasförmigen Inertstoff (**3**), wobei die gleichen Inertstoffe geeignet sind, wie sie weiter oben für die optional erfolgende Verdünnung des Phosgens im Gasstrom (**10**) beschrieben wurden.

Die Aufrechterhaltung des Inertgasstroms (**30**) in Schritt (II) kann geschehen, indem (**a**) ein bei Raumtemperatur und Normaldruck bevorzugt flüssig vorliegender Inertstoff (**3**) einer Temperatur T₃, welche kleiner als die für den Inertgasstrom (**30**) in Schritt (II) angestrebte Temperatur ist, in die Vorrichtung **1000** eingeleitet, dort erhitzt und anschließend in die Einrichtung **1100** und die anderen zu inertisierenden Anlagenteile geleitet wird. Bevorzugt beträgt die Temperatur des Inertgasstroms (**30**) von Schritt (II) 200 °C bis 600 °C, besonders bevorzugt 200 °C bis 500 °C und ganz besonders bevorzugt 250 °C bis 500 °C. Diese Ausführungsform ist insbesondere dann vorteilhaft, wenn das Reaktionsgemisch während der Reaktion im Regelbetrieb mit den Dämpfen eines bei Raumtemperatur und Normaldruck flüssig vorliegenden Inertstoffs (**3**) verdünnt wird. Es ist möglich, den Inertstoff (**3**) flüssig in die Vorrichtung **1000** einzutragen und dort erst zu verdampfen. In der Variante (a) besonders geeignete Inertstoffe (**3**) sind inerte Lösungsmittel wie aromatische Kohlenwasserstoffe, ggf. mit Halogensubstitution, wie z. B. Chlorbenzol oder Dichlorbenzol (alle Isomere, bevorzugt *ortho*-Dichlorbenzol). In der Vorrichtung **1000** wird der Inertstoff (**3**) so erhitzt (d. h. bei Eintrag als Flüssigkeit verdampft), dass ein Inertgasstrom (**30**) erhalten wird, der eine Temperatur T₃₀ von bevorzugt 200 °C bis 600 °C, besonders bevorzugt 200 °C bis 500 °C und ganz besonders bevorzugt 250 °C bis 500 °C, und einen absoluten Druck p₃₀ von bevorzugt 100 mbar bis 3.000 mbar, besonders bevorzugt 150 mbar bis 2.800 mbar und ganz besonders bevorzugt 200 mbar bis 2.500 mbar, aufweist. Die Ströme (**3**) und (**30**) unterscheiden sich nicht in ihrer chemischen Zusammensetzung, sondern lediglich in Temperatur und ggf. Druck. Nachdem der in die Vorrichtung **1000** eingetragene Strom (**3**) erhitzt wurde, so dass er die Vorrichtung **1000** gasförmig verlässt, wird er als Strom (**30**) bezeichnet. Wird Schritt (I) gemäß Variante (a) ausgeführt, so ist es bevorzugt, auch Schritt (II) gemäß Variante (a) auszuführen.

Es ist **(b)** auch möglich, in die Vorrichtung **1000** direkt einen Inertgasstrom (**30**) einer Temperatur T₃₀ von bevorzugt 200 °C bis 600 °C, besonders bevorzugt 200 °C bis 500 °C und ganz besonders bevorzugt 250 °C bis 500 °C, und eines absoluten Drucks p₃₀ von bevorzugt 100 mbar bis 3.000 mbar, besonders bevorzugt 150 mbar bis 2.800 mbar und ganz besonders bevorzugt 200 mbar bis 2.500 mbar, einzuleiten. Es ist in dieser Ausführungsform auch möglich, den eingeleiteten Inertgasstrom (**30**) in der Vorrichtung **1000** im Rahmen der zuvor definierten Temperaturbereiche weiter aufzuheizen. Alternativ kann der Inertgasstrom (**30**) auch direkt in die Einrichtung **1100** eingespeist werden. Variante (b), egal in welcher Ausgestaltung, ist insbesondere dann vorteilhaft, wenn das Reaktionsgemisch während der Reaktion im Regelbetrieb mit einem bei Raumtemperatur und Normaldruck bereits gasförmig vorliegenden Inertstoff wie Stickstoff, Helium oder Argon verdünnt wird. Wird Schritt (I) gemäß Variante (b) ausgeführt, so ist es bevorzugt, auch Schritt (II) gemäß Variante (b) auszuführen.

Die Durchführung des Schrittes (II) erst nach Verstreichen eines Zeitraums, der mindestens der 10-fachen Verweilzeit, bevorzugt mindestens der 30-fachen Verweilzeit, besonders bevorzug mindestens der 60-fachen Verweilzeit, ganz besonders bevorzugt mindestens der 90-fachen Verweilzeit von Phosgen (**1**) von Austritt aus der Vorrichtung **1000** bis Austritt aus der Reaktionsabbruchzone (**4000**) im Regelbetrieb der Gasphasenphosgenierungsanlage (**100**) entspricht, gerechnet ab dem Zeitpunkt, ab dem M'**(2)** null beträgt, bewirkt, dass Restmengen an noch nicht umgesetztem Amin (**2**) phosgeniert werden können.

In einer bevorzugten Ausgestaltung umfasst die Gasphasenphosgenierungsanlage (**100**) den Aufarbeitungsteil (**5000**). Dann ist es besonders bevorzugt im Regelbetrieb der Gasphasenphosgenierungsanlage (**100**) als Phosgen (**1**) im Phosgengasstrom (**10**) eine Mischung aus Frisch-Phosgen (**1'**) und in den Einrichtungen **5100** zurückgewonnenem Rezyklat-Phosgen (**1"**) einzusetzen. Diese Verfahrensführung erlaubt ferner eine besonders vorteilhafte Ausgestaltung des Schrittes (II) des erfindungsgemäßen Verfahrens dergestalt, dass nach erfolgter Reduktion des Aminmassenstroms M'**(2)** auf null,
(II.a) zunächst die Zufuhr von Frisch-Phosgen (**1'**) unterbrochen wird, und dann
(II.b) erst nach Verstreichen eines Zeitraums, der mindestens der 10-fachen Verweilzeit, bevorzugt mindestens der 30-fachen Verweilzeit, besonders bevorzug mindestens der 60-fachen Verweilzeit, ganz besonders bevorzugt mindestens der 90-fachen Verweilzeit von Phosgen (**1**) von Austritt aus der Vorrichtung **1000** bis Austritt aus der Reaktionsabbruchzone (**4000**) im Regelbetrieb der Gasphasenphosgenierungsanlage (**100**) entspricht, gerechnet ab dem Zeitpunkt, ab dem M'(**2**) null beträgt, auch die Zufuhr von Rezyklat-Phosgen (**1"**) unterbrochen wird, sodass der Massenfluss an Phosgen M'(**1**), welcher die Vorrichtung **1000** verlässt, null beträgt,
wobei während der Schritte (II.a) und (II.b), wie weiter oben beschrieben, ein gasförmiger Inertgasstrom (**30**) durch die Einrichtung **1100,** die Mischzone (**3100**), die Reaktionszone (**3200**) und die Reaktionsabbruchzone (4000), und optional, mindestens durch Teile der Einrichtung 5100, aufrechterhalten wird.

Unabhängig davon, ob die Gasphasenphosgenierungsanlage (100) den Aufarbeitungsteil 5000 umfasst, werden die Schritte (I) und (II) bevorzugt innerhalb von weniger als 12 Stunden, bevorzugt innerhalb von weniger als 6 Stunden, besonders bevorzugt innerhalb von weniger als 3 Stunden und ganz besonders bevorzugt innerhalb von weniger als 1 Stunde durchgeführt. Bevorzugt werden in den Schritten (I) und (II) Inertgasströme (30) identischer Zusammensetzung eingesetzt.

Der Inertgasstrom (**30**) aus (I) wird **(III)** mindestens für einen Zeitraum aufrechterhalten, welcher der dreifachen, bevorzugt der 100-fachen, besonders bevorzugt der 200-fachen, ganz besonders bevorzugt der 350-fachen, Verweilzeit des Inertgasstroms (**30**) von Eintritt in die Mischzone (**3100**) bis Austritt aus der Reaktionsabbruchzone (**4000**) entspricht, gerechnet ab dem Zeitpunkt, ab dem der Massenflusses an Phosgen M'(**1**), welcher die Vorrichtung **1000** verlässt, null beträgt. In einer bevorzugten Ausführungsform wird währenddessen auch der Inertgasstrom aus (II) aufrechterhalten, wobei dann die Verweilzeit der vereinigten Inertgasströme aus (I) und (II) maßgeblich ist. Im Anschluss an Schritt (III) ist es - nach Abkühlung - ggf. möglich, Anlagenteile für Wartungsarbeiten zu öffnen.

Die Inertgasströme (**30**) aus den Schritten (I) und (II) werden bevorzugt nach Austritt aus der Reaktionsabbruchzone (**4000**) oder, sofern vorhanden, bevorzugt nach Durchlaufen mindestens von Teilen der Aufarbeitungseinrichtungen **5100,** über eine Abgasausschleusung ausgetragen. In der bevorzugten Ausgestaltung des erfindungsgemäßen Verfahrens unter Einbeziehung des Aufarbeitungsteils (**5000**) durchlaufen die Inertgasströme (**30**) aus den Schritten (I) und (II) bevorzugt die weiter oben definierten Apparate Auswaschkolonne (**5110**) und Phosgenabsorptionskolonne (**5120**) und werden anschließend ausgeschleust.

Sollen zwei oder mehr Reaktionszonen (**3200**) parallel betrieben werden, ist es bevorzugt, jedoch nicht zwingend, diese nacheinander, wie vorstehend beschrieben, außer Betrieb zu nehmen. Die Nebensysteme (wie die HCl-Absorption, Phosgenabsorption, ggf. Lösungsmittelaufarbeitung oder auch die Abgasbehandlung) müssen so dimensioniert sein, dass die zum Abfahren benötigten Mengen an Inertgasströmen (**30**) gehandhabt werden können.

Fährt man alle Eduktwege gleichzeitig ab, dann können die oben beschriebenen Probleme auftreten. Phosgen kann in die Einrichtung **2100** (bevorzugt Amindüse) strömen und es kann zu Blockierungen, Anbackungen mit Polyharnstoff etc. kommen. Zusätzlich wird der in der kontinuierlichen Produktion bei Soll-Massenfluss M'_{Soll}(**2**) angestrebte Phosgenüberschuss zumindest kurzzeitig signifikant unterschritten, wobei wiederum Nebenprodukte entstehen, weil die Strömungsgleichgewichte gestört sind und es zu unkontrollierter Vermischung kommt. Die Verweilzeit der Edukte im Reaktionsraum wird gestört, wenn beide Eduktströme gleichzeitig abgeschaltet werden.

Durch die erfindungsgemäßen Vorgehensweisen bei der Außerbetriebnahme einer Gasphasenphosgenierungsanlage (**100**) ergeben sich daher die folgenden Vorteile für die Wiederinbetriebnahme:
i) Vermeidung von Verstopfungen in der Einrichtung **2100** (bevorzugt der Amindüse) und in der Mischzone (**3100**) und somit eine Vermeidung von sonst evtl. erforderlichem mehrfachem Anfahren, weil die Anlage zur Reinigung der Einrichtung **2100** (bevorzugt der Amindüse) abgefahren werden musste.
ii) Bedingt durch (i), Einsparung von Energie.
iii) Erhöhung der Produktivität der Gasphasen-Phosgenierungsanlage, weil nicht wegen der Entstehungen von Anbackungen und Ablagerungen mehrmals ab- und neu angefahren werden muss.
iv) Erhöhung der Produktivität und Minimierung an Verlusten von Einsatzstoffen der Phosgenerzeugung.
v) Vergleichmäßigung der Produktion und Erhöhung der Anlagensicherheit, weil die thermische Belastung der Phosgenierungsanlage (**100**) durch die Verringerung von Anfahrvorgängen reduziert wird.
vi) Verringerte Nebenproduktbildung sowie eine verkürzte thermische Belastung des Produktes und damit einhergehend eine Erhöhung der relativen Ausbeute.
vii) Vermeidung bzw. Verringerung von Niederschlägen, Anbackungen und Verstopfungen im Equipment (bspw. in der Vorrichtung **2000** und der Einrichtung **2100** oder in der Reaktionszone vor dem Reaktionsabbruch) und damit einhergehend eine Verlängerung der Standzeit des Verfahrens.
viii) Geringerer Abfall nach Reinigung des Equipments (bspw. weniger Polyharnstoff zu entfernen, Vermeidung von Waschwasser, das Korrosion auslösen könnte).
ix) Vermeidung von nicht spezifikationsgerechter Ware, die durch mehrfaches schlechtes An- und Abfahren entstehen kann. Solch eine qualitativ schlechte Anfahrware muss somit nicht mit qualitativ gutem Polyisocyanat verschnitten oder sogar im schlimmsten Fall verbrannt werden.

Somit ermöglicht das erfindungsgemäße Verfahren durch Vermeidung von Rückvermischungen von Phosgen (**1**) in den Amingasstrom (**20**) während der Außerbetriebnahme einer Gasphasenphosgenierungsanlage eine reibungslose Wiederinbetriebnahme der Anlage und die anschließende Aufarbeitung des entstandenen Roh-Isocyanats in technisch reibungsloser Weise mit verminderten oder im Idealfall ohne Ausfallzeiten mit direkt hoher Endproduktqualität. Aufwändige Reinigungsarbeiten während eines Stillstands können so vermieden oder zumindest im Umfang verringert werden.

### Beispiele

Gehaltsangaben in ppm oder % sind Massenanteile bezogen auf die Gesamtmasse des jeweiligen Stoff(strom)s.

### Allgemeine Bedingungen für die Herstellung von Toluylendiisocyanat (TDI) bei "eingefahrener" Gasphasen-Produktionsanlage (100) im Regelbetrieb

### (Siehe auch FIG. 1 (vereinfachte Darstellung))

Toluylendiamin (TDA) (**2**) wird in einem Aminverdampfer (**2000**) gemeinsam mit Stickstoff (**3**) kontinuierlich verdampft. Der so erhaltene Amingasstrom (**20**), enthaltend 12 t/h gasförmiges TDA (**2**), wird über eine Leitung (**2100**), an deren zum Phosgenierungsreakor (**3000**) hin gelegenen Ende sich eine Amindüse befindet, kontinuierlich in den Phosgenierungsreaktor (**3000**) eingedüst. Die Verweilzeit des TDA-Stroms (**20**) von Verlassen des Verdampfers (**2000**) bis zum Austritt aus der Amindüse beträgt dabei 5 Sekunden. Gleichzeitig werden über einen Phosgengleichrichter, der, wie in EP-A-1 362 847 offenbart, eingesetzt wird, 61 t/h eines gasförmigen Phosgenstroms (**10**) kontinuierlich in den Phosgenierungsreaktor (**3000**) eingedüst. Das eingesetzte Phosgen ist eine Mischung aus frischem Phosgen (**1**') und im Aufarbeitungsteil (**5000**) zurückgewonnenem Phosgen (**1"**). Dabei werden die beiden Edukte gut vermischt, und eine Rückvermischung unterbleibt. Die Temperatur des gasförmigen TDA-Stroms (**20**) am Düsenmund beträgt 380 °C (TDA hat ca. 1 Sekunde Verweilzeit bei dieser Temperatur im Zulauf zum Düsenmund). Das gasförmige Phosgen (**10**) hat eine Temperatur von 320 °C beim Verlassen des Phosgengleichrichters, wobei die Verweilzeit des heißen Phosgens zwischen letztem Phosgenüberhitzer und Phosgengleichrichter 2 Sekunden beträgt. Das gasförmige Gemisch aus den Strömen (**10**) und (**20**) hat eine Verweilzeit von 8 Sekunden im Gasphasenreaktor (**3000**) und reagiert bei einem absoluten Druck von 1.692 mbar zu gasförmigem Reaktionsgemisch (**40**). Die sich anschließende Reaktionsabbruchzone (**4000**) umfasst eine zweistufige "Quenche", in welcher das gasförmige Reaktionsgemisch (**40**) durch Einsprühen von ortho-Dichlorbenzol (ODB) auf 168 °C abgekühlt wird, sodass es kondensiert und sich im Sumpfbehälter (**4100**) ein Gemisch (**60**) aus Roh-TDI und ODB ansammelt. Überschüssiges Phosgen, bei der Reaktion entstandener Chlorwasserstoff und Inerte werden unter diesen Bedingungen aus dem Sumpfbehälter (**4100**) weitestgehend entgast, wobei der Mitriss an TDI mittels Einbauten vermindert wird. Dieser Prozessrestgasstrom (**50**) wird zur Wiedergewinnung von mitgerissenem TDI, Phosgen und Chlorwasserstoff, wie in WO 2011/003532, Seite 11, Zeile 24 bis 25 beschrieben, aufgearbeitet (**5100**). Das Gemisch (**60**) aus dem Sumpfbehälter (**4100**) wird, wie in EP 1 413 571 B1 beschrieben, aufgearbeitet (**5200**), wobei TDI (**4**) in einem Mengenstrom von 15,6 t/h erhalten wird.

So hergestelltes TDI (**4**) hat typischerweise eine Reinheit von > 99,97 % (Gaschromatographie, GC), einen Restlösemittelgehalt an ODB von < 5 ppm (GC), einen Restchlorgehalt von hydrolisierbarem Chlor von < 10 ppm (Titration gemäß ASTM D4663), eine Acidität von gebundenem Chlor < 5 ppm (Titration gemäß ASTM D5629), und die Farbzahl, gemessen als Hazenzahl, ist < 15 (bestimmt nach DIN EN ISO 6271).

### Vergleichsbeispiel 1: Außerbetriebnahme einer Gasphasenphosgenieranlage (100) unter Abstellen der Phosgenzufuhr vor der Aminzufuhr

Eine Gasphasenphosgenierungsanlage (**100**) wird, wie in den allgemeinen Bedingungen zur Herstellung von TDI beschrieben, bei einer Produktionskapazität von 15,6 t/h TDI betrieben und dann wie folgt außer Betrieb genommen:
Die Phosgenzufuhr wird unterbrochen, während die Aminzufuhr noch ca. 2 Minuten weiter aufrechterhalten wird. Das Amin TDA wird während dieser Zeit nicht mehr vollständig phosgeniert, und die entstehenden Zwischen- und Nebenprodukte verstopfen die Mischzone (**3100**), die Reaktionszone (**3200**) und die sich anschließende Reaktionsabbruchzone ("Quenche", **4000**). Der Differenzdruck von Eintritt der Edukte TDA-Gasstrom (**20**) und Phosgen-Gasstrom (**10**) in den Phosgenierreaktor (**3000**) über den Brüdengasaustritt des Sumpfes der Reaktionsabbruchzone (**4000**) bis zur TDI-Auswaschkolonne (**5110**) steigt innerhalb der kurzen Zeit, während der Phosgengasstrom (**10**) unterbrochen und der Amingasstrom (**20**) noch weiter lief (ca. 2 Minuten), auf 913 mbar, statt 10 mbar bei Normalbetrieb, an. Eine Reinigung des Reaktors und seiner Peripherie muss vorgenommen werden, bevor eine Wiederinbetriebnahme möglich ist.

### Vergleichsbeispiel 2: Außerbetriebnahme einer Gasphasenphosgenieranlage (100) unter gleichzeitigem Abstellen der Amin- und Phosgenzufuhr

Die Gasphasenanlage (**100**) wird, wie in den allgemeinen Bedingungen zur Herstellung von TDI beschrieben, bei einer Produktionskapazität von 15,6 t/h TDI betrieben und dann wie folgt außer Betrieb genommen:
Der Phosgengenerator wird abgestellt. Danach werden die Amin- und Phosgenzufuhr zum Phosgenierreaktor (**3000**) hin gleichzeitig unterbrochen. Anschließend wird der Phosgenierreaktor **(3000)** mit Stickstoff inertisiert, bis der Phosgenierreaktor und seine Rohrleitungsperipherie von Edukten und Produkt befreit sind.

Nach einer kurzen Reaktionsunterbrechung werden der Aminverdampfer inklusive nachgeschalteter Wärmeaustauscher (**2000**) und die Einrichtungen **2100** inklusive der Amindüse mit Stickstoff beaufschlagt, wobei eine Temperatur von 380 °C eingestellt wird. Der Phosgenierreaktor (**3000**) ist Edukt- und Produkt-frei und mit heißem Stickstoff inertisiert. Die Aminverdampfung im Aminverdampfer wird gestartet, sodass TDA bei 300 °C verdampft. Anschließend wird das TDA in weiteren Wärmeaustauschern stufenweise auf 380 °C erhitzt und als gasförmiger TDA-Strom (**20**) eines Drucks von 1.691 mbar (absolut) durch die Amindüse in den Phosgenierreaktor (**3000**) eingedüst. Innerhalb von 45 Minuten wird der Massenstrom an TDA (**2**), welcher in den Phosgenierreaktor (**3000**) eingeleitet wird, stufenlos von 0 t/h auf 12 t/h erhöht. Kurz vor dem Durchtritt des TDA-Gasstroms (**20**) durch die Amindüse in den Phosgenierreaktor (**3000**) wird der Phosgenweg geöffnet, und ein Phosgengasstrom (**10**) wird mit einem Massenfluss von 61 t/h und einer Temperatur von 320 °C und einem Druck von 1691 mbar (absolut) am Reaktoreintritt in den Phosgenierreaktor (**3000**) eingedüst. Die Anlage erreicht nach 45 Minuten den Regelbetrieb-Zustand. Nach 7 Tagen muss die Anlage abgestellt werden, weil der Differenzdruck von Eintritt der Edukte TDA-Gasstrom (**20**) und Phosgen-Gasstrom (**10**) in den Phosgenierreaktor (**3000**) über den Brüdengasaustritt am Sumpf der Reaktionsabbruchzone (**4000**) bis zur TDI-Auswaschkolonne (**5110**) über die Zeit auf 793 mbar, statt 10 mbar bei Normalbetrieb, angestiegen ist, und die benötigte Energie zum Verdampfen von Phosgen und TDA und Überführung der Gasströme (**10**) und (**20**) in den Phosgenierreaktor (**3000**) kaum noch aufgebracht werden kann (die Siedetemperatur des TDA (**2**) limitiert bei zunehmendem Druck die Verdampferkapazität). Nach Abstellung und Öffnen der Anlage werden am Austritt der Amindüse, entlang der Oberfläche des Reaktorraumes und auf den Oberflächen der Quenchen Polyharnstoff-haltige massive Ablagerungen gefunden.

### Vergleichsbeispiel 3: Außerbetriebnahme einer Gasphasenphosgenieranlage (100) unter Abstellen der Aminzufuhr vor der Phosgenzufuhr, jedoch ohne Inertgaspülung der Aminzuführungseinrichtungen (Rückströmen Phosgen in Amindüse)

Eine Gasphasenproduktionsanlage (**100**) wird, wie in den allgemeinen Bedingungen zur Herstellung von TDI beschrieben, bei einer Produktionskapazität von 15,6 t/h TDI betrieben und dann wie folgt außer Betrieb genommen:
Die Aminzufuhr zum Phosgenierreaktor (**3000**) wird unterbrochen, indem die Edukt-führende Rohrleitung (Bestandteil der Einrichtung **2100**) hinter dem Aminverdampfer (**2000**) geschlossen wird. Eine Inertgasspülung der Einrichtung **2100** wird nicht durchgeführt. Danach wird der Phosgengenerator abgestellt und so die Zufuhr von Frisch-Phosgen (**1**') unterbrochen. Nach 15 Minuten (entsprechend ca. der 112-fachen Verweilzeit von Phosgen (**1**) von Austritt aus der Vorrichtung **1000** bis Austritt aus der Reaktionsabbruchzone (**4000**) im Regelbetrieb der Gasphasenphosgenierungsanlage **(100))** wird der Phosgenkreislauf heruntergefahren, indem auch die Zufuhr von Rezyklat-Phosgen (**1"**) zum Phosgenierreaktor (**3000**) unterbrochen wird. Anschließend wird der Phosgenierreaktor (**3000**) mit Stickstoff inertisiert.

Nach einem Kurzstillstand wird ein Phosgenkreislauf aufgebaut, indem Rezyklat-Phosgen (**1"**) aus der Aufarbeitung (**5100**) der Brüden (**50**) der Reaktionsabbruchzone (**4000**) mit einer Temperatur von 320 °C über den Phosgenierungsreaktor (**3000**), die Reaktionsabbruchzone (**4000**) zurück zur Aufarbeitung gefahren wird. In der Reaktionsabbruchzone (**4000**) ist währenddessen nur die in Strömungsrichtung des Reaktionsgemisches (**40**) zweite Quenche in Betrieb, wodurch der Phosgenstrom abgekühlt wird. In diesem Phosgenkreislauf werden 61 t/h Phosgen umgewälzt. Die Leitung **2100** einschließlich der Amindüse werden währenddessen mit einem Stickstoffgasstrom (**30**) gespült. Sobald der Phosgenierungsreaktor (**3000**) auf 320 °C temperiert ist, wird die Aminverdampfung gestartet indem auf 220 °C vorgeheiztes, flüssiges TDA (**2**) gemeinsam mit Stickstoff in den Aminverdampfer (**2000**) gefahren wird, dort mit Hilfe eines Wärmeaustauschers bei 300 °C verdampft und anschließend mit einem weiteren Wärmeaustauscher auf 410 °C erhitzt wird. Der so erhaltene TDA-Strom (**20**) wird bei einem absoluten Druck von 1654 mbar durch die Amindüse in den Phosgenierungsreaktor (**3000**) eingedüst. Die Menge an TDA (**2**), die während der Inbetriebnahme der Gasphasenphosgenierungsanlage (d. h. bis der Amin-Massenfluss M'Soll(**2**) erreicht ist, was nach 45 Minuten der Fall ist) in den Phosgenierungsreaktor (**3000**) eingeleitet wird, wird stufenlos von 0 t/h auf 12 t/h erhöht, wobei der Betriebsdruck im Phosgenierungsreaktor (**3000**) am Ende der Inbetriebnahme 1641 mbar (absolut) beträgt. Die in Strömungsrichtung des Reaktionsgemisches (**40**) erste Quenche wird in Betrieb genommen kurz bevor erstmalig TDA-Gasstrom (**20**) bei der Inbetriebnahme die Amindüse in Richtung des Phosgenierungsreaktors (**3000**) verlässt. Das nach dem Start der Anlage verbrauchte Phosgen wird durch eine Mischung aus frischem Phosgen (**1'**) und in der Aufarbeitung wiedergewonnenem Phosgen (**1**") ersetzt. Nach 60 Minuten verlassen 15,6 t/h an TDI (**4**) die letzte Destillationskolonne der Aufarbeitungsstufe.

Nach dem Anfahren steigt der Differenzdruck zwischen Aminverdampfung (**2000**) und Phosgenierungsreaktor (**3000**) immer weiter an. Nach 3 Stunden muss der Phosgenierungsreaktor (**3000**) abgestellt werden, weil der Betriebsdruck an der Amindüse auf 2,5 bar (absolut) gestiegen ist. Nach Abstellung und Öffnen der Anlage werden in der Austrittsöffnung der Amindüse und in der zur Amindüse führenden Rohrleitung verkohlte Rückstände gefunden. Dies ist auf Rückströmen von Phosgen in die Einrichtung **2100** während der Anfahrphase zurückzuführen, wobei TDI-Ablagerungen entstehen, welche die Austrittsöffnung der Amindüse verstopfen.

### Vergleichsbeispiel 4: Außerbetriebnahme einer Gasphasenphosgenieranlage (100) unter Abstellen der Aminzufuhr (unter Beibehaltung der Inertgaspülung der Aminzuführungseinrichtungen (Verhinderung Rückströmen Phosgen in Amindüse) vor der Phosgenzufuhr, jedoch mit zu kurzer Nachreaktionszeit (Verweilzeit) mit Phosgen

Die Gasphasenphosgenierungsanlage (**100**) wird, wie in den allgemeinen Bedingungen zur Herstellung von TDI beschrieben, bei einer Produktionskapazität von 15,6 t/h TDI betrieben und wie folgt außer Betrieb genommen:
Die Aminzufuhr zum Phosgenierreaktor (**3000**) wird unterbrochen, indem die Edukt-führende Rohrleitung (Bestandteil der Einrichtung **2100**) hinter dem Aminverdampfer (**2000**) geschlossen wird. Währenddessen wird ein gasförmiger Stickstoffstrom durch die Einrichtung **2100**, die Mischzone (**3100**), die Reaktionszone (**3200**), die Reaktionsabbruchzone (**4000**) und Teile der Einrichtung **5100** (Auswaschkolonne **5120** und Phosgenabsorptionskolonne **5120**, beide in FIG. 1 nicht gezeigt) aufrechterhalten (Schritt (I) des erfindungsgemäßen Verfahrens). Nach erfolgter Reduzierung des Amin-Massenflusses M'(**2**) auf null wird der Phosgengenerator abgestellt, und so die Zufuhr von Frischphosgen (**1'**) unterbrochen (Schritt (II.a) des erfindungsgemäßen Verfahrens). Nach 30 Sekunden (entsprechend ca. der 4-fachen Verweilzeit von Phosgen (1) von Austritt aus der Vorrichtung **1000** bis Austritt aus der Reaktionsabbruchzone (**4000**) im Regelbetrieb der Gasphasenphosgenierungsanlage **(100))** wird der Phosgenkreislauf heruntergefahren, indem auch die Zufuhr von Rezyklat-Phosgen (**1"**) zum Phosgenierreaktor (**3000**) unterbrochen wird (Schritt (II.b) des erfindungsgemäßen Verfahrens). Während der ganzen Zeit werden die Einrichtung **1100**, der Reaktor (**3000**), die Reaktionsabbruchzone (**4000**) und Teile der Einrichtung **5100** (Auswaschkolonne **5120** und Phosgenabsorptionskolonne **5120,** beide in FIG. 1 nicht gezeigt) mit Stickstoff durchspült, welcher die Einrichtung **5100** durch Ausschleusung aus der Phosgenabsorptionskolonne (**5120**) verlässt. Die Stickstoffströme aus den Schritten (I), (II.a) und (II.b) werden nach Unterbrechung des Phosgenkreislaufs noch 1 Stunde (entsprechend ca. dem 400-fachen der Verweilzeit der vereinigten Inertgasströme (**30**) von Eintritt in die Mischzone (**3100**) bis Austritt aus der Reaktionsabbruchzone **(4000))** aufrechterhalten (Schritt (III) des erfindungsgemäßen Verfahrens), nach deren Verlauf der Phosgenierreaktor (3000) von den Edukten und Produkt befreit ist.

Nach einem eintägigen Wartungsstillstand in einem anderen Teil der Anlage wird die Gasphasenphosgenierungsanlage, wie in Beispiel 3 beschrieben, wieder in Betrieb genommen.

Am Ende der Inbetriebnahme, also nach dem Anfahren, beträgt der Betriebsdruck im Phosgenierungsreaktor (3000) 1651 mbar (absolut). Nach 13 Tagen muss die Anlage abgestellt werden, weil der Differenzdruck von Eintritt der Edukte TDA-Gasstrom (20) und Phosgen-Gasstrom (**10**) in den Phosgenierreaktor (**3000**) über den Brüdengasaustritt am Sumpf der Reaktionsabbruchzone (**4000**) bis zur TDI-Auswaschkolonne (**5110**) über die Zeit auf 803 mbar, statt 10 mbar bei Normalbetrieb, angestiegen ist, und die benötigte Energie zum Verdampfen von Phosgen und TDA und Überführung der Gasströme (**10**) und (**20**) in den Phosgenierreaktor (**3000**) kaum noch aufgebracht werden kann (die Siedetemperatur des TDA (**2**) limitiert bei zunehmendem Druck die Verdampferkapazität). Nach Abstellung und Öffnen der Anlage werden am Austritt der Amindüse, entlang der Oberfläche des Reaktorraumes und auf den Oberflächen der Quenchen Polyharnstoff-haltige massive Ablagerungen gefunden.

### Beispiel 5 (erfindungsgemäß)

Die Gasphasenphosgenierungsanlage (**100**) wird, wie in den allgemeinen Bedingungen zur Herstellung von TDI beschrieben, bei einer Produktionskapazität von 15,6 t/h TDI betrieben und wie folgt außer Betrieb genommen:
Die Aminzufuhr zum Phosgenierreaktor (**3000**) wird unterbrochen, indem die Edukt-führende Rohrleitung (Bestandteil der Einrichtung **2100**) hinter dem Aminverdampfer (**2000**) geschlossen wird. Währenddessen wird ein gasförmiger Stickstoffstrom durch die Einrichtung **2100**, die Mischzone (**3100**), die Reaktionszone (**3200**), die Reaktionsabbruchzone (**4000**) und Teile der Einrichtung **5100** (Auswaschkolonne **5120** und Phosgenabsorptionskolonne **5120**, beide in FIG. 1 nicht gezeigt) aufrechterhalten (Schritt (I) des erfindungsgemäßen Verfahrens). Nach erfolgter Reduzierung des Amin-Massenflusses M'**(2)** auf null wird der Phosgengenerator abgestellt, und so die Zufuhr von Frischphosgen **(1')** unterbrochen (Schritt (II.a) des erfindungsgemäßen Verfahrens). Nach 15 Minuten (entsprechend ca. der 112-fachen Verweilzeit von Phosgen (**1**) von Austritt aus der Vorrichtung **1000** bis Austritt aus der Reaktionsabbruchzone (**4000**) im Regelbetrieb der Gasphasenphosgenierungsanlage **(100)**) wird der Phosgenkreislauf heruntergefahren, indem auch die Zufuhr von Rezyklat-Phosgen (**1"**) zum Phosgenierreaktor (**3000**) unterbrochen wird (Schritt (II.b) des erfindungsgemäßen Verfahrens). Während der ganzen Zeit werden die Einrichtung **1100,** der Reaktor (3000), die Reaktionsabbruchzone (**4000**) und Teile der Einrichtung **5100** (Auswaschkolonne **5120** und Phosgenabsorptionskolonne **5120,** beide in FIG. 1 nicht gezeigt) mit Stickstoff durchspült, welcher die Einrichtung **5100** durch Ausschleusung aus der Phosgenabsorptionskolonne (**5120**) verlässt. Die Stickstoffströme aus den Schritten (I), (II.a) und (II.b) werden nach Unterbrechung des Phosgenkreislaufs noch 1 Stunde (entsprechend ca. dem 400-fachen der Verweilzeit der vereinigten Inertgasströme (**30**) von Eintritt in die Mischzone (**3100**) bis Austritt aus der Reaktionsabbruchzone **(4000))** aufrechterhalten (Schritt (III) des erfindungsgemäßen Verfahrens), nach deren Verlauf der Phosgenierreaktor (**3000**) von den Edukten und Produkt befreit ist.

Nach einem eintägigen Wartungsstillstand in einem anderen Teil der Anlage wird die Gasphasenphosgenierungsanlage, wie in Beispiel 3 beschrieben, wieder in Betrieb genommen.

In Beispiel 5 werden keine Verblockung der Amindüse und keine Entstehung von Ablagerungen im Phosgenierreaktor während der Außerbetriebnahme beobachtet. Die Anlage kann ohne Probleme wieder in Betrieb genommen und über einen weiteren Phosgenierzyklus von mehreren Monaten betrieben werden. Die Entstehung von unerwünschten Nebenprodukten wie Polyharnstoffen etc. ist signifikant reduziert, und die Anfahrware weist hohe Qualität auf, sodass diese nicht mit zu einem späteren Zeitpunkt produziertem TDI verschnitten werden muss.

## Patentansprüche

1. Verfahren zum Betreiben einer Gasphasenphosgenierungsanlage (**100**) zur Umsetzung eines Amins (**2**) mit Phosgen (**1**) im stöchiometrischen Überschuss in Bezug auf die primären Aminogruppen des Amins (**2**) zum korrespondierenden Isocyanat (**4**), wobei die Gasphasenphosgenierungsanlage (**100**) mindestens
(i) eine Vorrichtung **1000** zur Bereitstellung eines gasförmigen Phosgenstroms (**10**), optional umfassend neben Phosgen (**1**) einen Inertstoff (**3**),
(ii) eine Vorrichtung **2000** zur Bereitstellung eines gasförmigen Aminstroms (**20**), optional umfassend neben Amin (**2**) einen Inertstoff (**3**),
(iii) eine Mischzone (**3100**) zur Vermischung der Ströme (**10**) und (**20**), wobei die Mischzone durch Einrichtungen (**1100**, **2100**) jeweils mit der Vorrichtung **1000** und der Vorrichtung **2000** verbunden ist,
(iv) eine strömungstechnisch nach der Mischzone (**3100**) angeordnete Reaktionszone (**3200**) zur weiteren Umsetzung der zuvor vermischten Ströme (**10**) und (**20**),
(v) eine strömungstechnisch nach der Reaktionszone (**3200**) angeordnete Reaktionsabbruchzone (**4000**) zur Beendigung der Umsetzung,
und optional
(vi) einen Aufarbeitungsteil (**5000**) umfassend Einrichtungen zur Rückgewinnung und Rückführung nicht umgesetzten Phosgens (**1"**) (**5100**) und Einrichtungen zur Gewinnung des hergestellten Isocyanats in Reinform (**5200**)
umfasst,
**dadurch gekennzeichnet, dass**
zur Außerbetriebnahme der Gasphasenphosgenierungsanlage (**100**) die folgenden Schritte durchlaufen werden:
(I) Reduzierung des Amin-Massenflusses M'(**2**) auf null, wobei ein gasförmiger Inertgasstrom (**30**) durch die Einrichtung **2100**, die Mischzone (**3100**), die Reaktionszone (**3200**) und die Reaktionsabbruchzone (**4000**) aufrechterhalten wird;
(II) erst nach Verstreichen eines Zeitraums, der mindestens der 10-fachen Verweilzeit, bevorzugt mindestens der 30-fachen Verweilzeit, besonders bevorzugt mindestens der 60-fachen Verweilzeit, ganz besonders bevorzugt mindestens der 90-fachen Verweilzeit von Phosgen (**1**) von Austritt aus der Vorrichtung **1000** bis Austritt aus der Reaktionsabbruchzone (**4000**) im Regelbetrieb der Gasphasenphosgenierungsanlage (**100**) entspricht, gerechnet ab dem Zeitpunkt, ab dem M'(**2**) null beträgt:
Reduzierung des Massenflusses an Phosgen M'(**1**), welcher die Vorrichtung **1000** verlässt, auf null, wobei ein gasförmiger Inertgasstrom (**30**) durch die Einrichtung **1100,** die Mischzone (**3100**), die Reaktionszone (**3200**) und die Reaktionsabbruchzone (**4000**) aufrechterhalten wird;
(III) Aufrechterhalten zumindest des Inertgasstroms (**30**) aus (I), bevorzugt der Inertgasströme (**30**) aus (I) und (II), mindestens für einen Zeitraum, welcher der dreifachen, bevorzugt der 100-fachen, besonders bevorzugt der 200-fachen, ganz besonders bevorzugt der 350-fachen Verweilzeit des Inertgasstroms (**30**) von Eintritt in die Mischzone (**3100**) bis Austritt aus der Reaktionsabbruchzone (**4000**) entspricht, gerechnet ab dem Zeitpunkt, ab dem der Massenfluss an Phosgen M'(**1**), welcher die Vorrichtung **1000** verlässt, null beträgt.

2. Verfahren nach Anspruch 1, bei dem die Gasphasenphosgenierungsanlage (**100**) den Aufarbeitungsteil (**5000**) umfasst und bei dem im Regelbetrieb der Gasphasenphosgenierungsanlage (**100**) das Phosgen (**1**) im Phosgengasstrom (**10**) eine Mischung aus Frisch-Phosgen (**1**') und in den Einrichtungen **5100** zurückgewonnenem Rezyklat-Phosgen (**1"**) ist.

3. Verfahren nach Anspruch 2, bei dem nach erfolgter Reduzierung des Amin-Massenflusses M'(**2**) auf null
(II.a) zunächst die Zufuhr von Frisch-Phosgen (**1'**) unterbrochen wird, und dann
(II.b) erst nach Verstreichen eines Zeitraums, der mindestens der 10-fachen Verweilzeit, bevorzugt mindestens der 30-fachen Verweilzeit, besonders bevorzug mindestens der 60-fachen Verweilzeit, ganz besonders bevorzugt mindestens der 90-fachen Verweilzeit von Phosgen (**1**) von Austritt aus der Vorrichtung **1000** bis Austritt aus der Reaktionsabbruchzone **(4000)** im Regelbetrieb der Gasphasenphosgenierungsanlage (**100**) entspricht, gerechnet ab dem Zeitpunkt, ab dem M'(**2**) null beträgt, auch die Zufuhr von Rezyklat-Phosgen (**1"**) unterbrochen wird, sodass der Massenfluss an Phosgen M'(**1**), welcher die Vorrichtung **1000** verlässt, null beträgt,
wobei während (II.a) und (II.b) ein gasförmiger Inertgasstrom (**30**) durch die Einrichtung **1100,** die Mischzone **(3100),** die Reaktionszone **(3200)** und die Reaktionsabbruchzone **(4000)** aufrechterhalten wird.

4. Verfahren nach einem der Ansprüche 1 bis 3, bei dem die Zusammensetzungen und Massenflüsse der Ströme **10** und **20** bei Betrieb der Gasphasenphosgenierungsanlage (**100**) im Regelbetrieb so aufeinander abgestimmt werden, dass in Strom **10** Phosgen (**1**) in einem stöchiometrischen Überschuss in Bezug auf die primären Aminogruppen des Amins (**2**) in Strom **20** von mindestens 150 % der Theorie vorliegt.

5. Verfahren nach einem der Ansprüche 1 bis 4, bei dem in Schritt (I) der Amin-Massenfluss M'(**2**) stufenlos oder in Stufen, bevorzugt stufenlos, auf null reduziert wird.

6. Verfahren nach einem der Ansprüche 2 bis 5, bei dem in Schritt (II.a) die Zufuhr des Frisch-Phosgens (**1'**) stufenlos oder in Stufen, bevorzugt stufenlos, auf null reduziert wird.

7. Verfahren nach einem der Ansprüche 1 bis 6, bei dem die Einrichtung **1100** eine Düse umfasst.

8. Verfahren nach einem der Ansprüche 1 bis 7, bei dem die Einrichtung **2100** eine Düse umfasst.

9. Verfahren nach einem der Ansprüche 1 bis 6, bei dem die Einrichtungen **1100** und **2100** eine gemeinsame Düsenvorrichtung umfassen.

10. Verfahren nach einem der Ansprüche 1 bis 9, bei dem die Reaktionsabbruchzone (**4000**) durch Kontaktieren des aus der Reaktionszone (**3200**) austretenden gasförmigen Verfahrensprodukts mit einem inerten Lösungsmittel unterhalb der Siedetemperatur des Isocyanats (**4**) und oberhalb der Zersetzungstemperatur des zum Amin (**2**) korrespondieren Carbamoylchlorids betrieben wird.

11. Verfahren nach Anspruch 10, bei dem die Reaktionsabbruchzone (**4000**) frühestens dann außer Betrieb genommen wird, wenn der Massenfluss an Phosgen M'(**1**), welcher die Vorrichtung **1000** verlässt, null beträgt.

12. Verfahren nach einem der Ansprüche 1 bis 11, bei dem der Inertgasstrom (**30**) in Schritt (I) in die Vorrichtung **2000** oder in die Einrichtung **2100** eingeleitet wird und bei dem der Inertgasstrom (**30**) in Schritt (II) in die Vorrichtung **1000** oder die Einrichtung **1100** eingeleitet wird.

13. Verfahren nach einem der Ansprüche 1 bis 11, bei dem der Inertgasstrom (**30**) in Schritt (I) durch Erhitzen eines in die Vorrichtung **2000** eingetragenen Inertstoffs (**3**), der bevorzugt bei Raumtemperatur und Normaldruck flüssig ist, erhalten wird, und bei dem der Inertgasstrom (**30**) in Schritt (II) durch Erhitzen eines in die Vorrichtung **1000** eingetragenen Inertstoffs (**3**), der bevorzugt bei Raumtemperatur und Normaldruck flüssig ist, erhalten wird.

14. Verfahren nach einem der Ansprüche 1 bis 13, bei dem die Inertgasströme (**30**) in Schritt (I) und in Schritt (II) unabhängig voneinander eine Temperatur im Bereich von 200 °C bis 600 °C aufweisen.

15. Verfahren nach einem der Ansprüche 1 bis 14, bei dem das Amin (**2**) ausgewählt ist aus der Gruppe bestehend aus Isophorondiamin, Hexamethylendiamin, Bis(p-aminocyclohexyl)methan, Toluylendiamin und Diphenylmethandiamin.

## Claims

1. Method of operating a gas phase phosgenation plant (**100**) for reacting an amine (**2**) with phosgene **(**1) in a stoichiometric excess in relation to the primary amino groups of the amine (**2**) to give the corresponding isocyanate (**4**), said gas phase phosgenation plant (**100**) comprising at least
(i) an apparatus **1000** for providing a gaseous phosgene stream (**10**), optionally comprising, as well as phosgene (**1**), an inert substance (**3**),
(ii) an apparatus **2000** for providing a gaseous amine stream (**20**), optionally comprising, as well as amine (**2**), an inert substance (**3**),
(iii) a mixing zone (**3100**) for mixing the streams (**10**) and (**20**), the mixing zone being connected by each of devices (**1100, 2100**) to the apparatus **1000** and the apparatus **2000,**
(iv) a reaction zone (**3200**) arranged downstream of the mixing zone (**3100**) for further conversion of the previously mixed streams (**10**) and (**20**),
(v) a reaction stopping zone (**4000**) arranged downstream of the reaction zone (**3200**) to end the reaction, and optionally
(vi) a workup section (**5000**) comprising devices for recovery and recyling of unconverted phosgene (**1"**) (**5100**) and devices for obtaining the isocyanate prepared in pure form (**5200**),
**characterized in that**
the gas phase phosgenation plant (**100**) is shut down by running the following steps:
(I) reducing the amine mass flow rate M' (**2**) to zero, while maintaining a gaseous inert gas stream (**30**) through the device **2100,** the mixing zone (**3100**), the reaction zone (**3200**) and the reaction stopping zone (**4000**);
(II) only after a period corresponding to at least 10 times the residence time, preferably at least 30 times the residence time, more preferably at least 60 times the residence time and most preferably at least 90 times the residence time of phosgene (**1**) from exit from the apparatus **1000** to exit from the reaction stopping zone (**4000**) in the regular operation of the gas phase phosgenation plant (**100**) has elapsed, calculated from the moment from which M'(**2**) is zero:
reducing the mass flow rate of phosgene M'(**1**) leaving the apparatus **1000** to zero, while maintaining a gaseous inert gas stream (**30**) through the device **1100,** the mixing zone (**3100**), the reaction zone (**3200**) and the reaction stopping zone (**4000**);
(III) maintaining at least the inert gas stream (**30**) from (I), preferably the inert gas streams (**30**) from (I) and (II), at least for a period corresponding to three times, preferably 100 times, more preferably 200 times and most preferably 350 times the residence time of the inert gas stream (**30**) from entry into the mixing zone (**3100**) to exit from the reaction stopping zone (**4000**), calculated from the moment from which the mass flow rate of phosgene M'(**1**) leaving the apparatus **1000** is zero.

2. Method according to Claim 1, in which the gas phase phosgenation plant (**100**) includes the workup section (**5000**) and in which, in the regular operation of the gas phase phosgenation plant (**100**), the phosgene (**1**) in the phosgene gas stream (**10**) is a mixture of fresh phosgene (**1'**) and recycled phosgene (**1"**) recovered in the devices **5100.**

3. Method according to Claim 2, in which, after the amine mass flow rate M'(**2**) has been reduced to zero,
(II.a) first the supply of fresh phosgene (**1'**) is stopped, and then
(II.b) only after a period corresponding to at least 10 times the residence time, preferably at least 30 times the residence time, more preferably at least 60 times the residence time and most preferably at least 90 times the residence time of phosgene (**1**) from exit from the apparatus **1000** to exit from the reaction stopping zone **(4000)** in the regular operation of the gas phase phosgenation plant (**100**) has elapsed, calculated from the moment from which M'(**2**) is zero, the supply of recycled phosgene (**1"**) is also stopped, such that the mass flow rate of phosgene M' (**1**) leaving the apparatus **1000** is zero,
with maintenance during (II.a) and (II.b) of a gaseous inert gas stream (**30**) through the device **1100**, the mixing zone (**3100**), the reaction zone (**3200**) and the reaction stopping zone (**4000**).

4. Method according to any of Claims 1 to 3, in which the compositions and mass flow rates of streams **10** and **20** in operation of the gas phase phosgenation plant (**100**) in regular operation are matched to one another such that phosgene (**1**) is present in stream **10** in a stoichiometric excess in relation to the primary amino groups of the amine (**2**) in stream **20** of at least 150% of theory.

5. Method according to any of Claims 1 to 4, in which, in step (I), the amine mass flow rate M'(**2**) is reduced to zero continuously or in stages, preferably continuously.

6. Method according to any of Claims 2 to 5, in which, in step (II.a), the supply of fresh phosgene (**1'**) is reduced to zero continuously or in stages, preferably continuously.

7. Method according to any of Claims 1 to 6, in which the device **1100** comprises a nozzle.

8. Method according to any of Claims 1 to 7, in which the device **2100** comprises a nozzle.

9. Method according to any of Claims 1 to 6, in which the devices **1100** and **2100** comprise a common nozzle apparatus.

10. Method according to any of Claims 1 to 9, in which the reaction stopping zone (**4000**) is operated below the boiling temperature of the isocyanate (**4**) and above the breakdown temperature of the carbamoyl chloride corresponding to the amine (**2**) by contacting the gaseous process product exiting from the reaction zone (**3200**) with an inert solvent.

11. Method according to Claim 10, in which the reaction stopping zone (**4000**) is taken out of operation no earlier than when the mass flow rate of phosgene M'(**1**) leaving the apparatus **1000** is zero.

12. Method according to any of Claims 1 to 11, in which the inert gas stream (**30**) in step (I) is introduced into the apparatus **2000** or into the device **2100,** and in which the inert gas stream (**30**) in step (II) is introduced into the apparatus **1000** or the device **1100.**

13. Method according to any of Claims 1 to 11, in which the inert gas stream (**30**) in step (I) is obtained by heating an inert substance (**3**) which is preferably liquid at room temperature and standard pressure and is introduced into the apparatus **2000,** and in which the inert gas stream (**30**) in step (II) is obtained by heating an inert substance (**3**) which is preferably liquid at room temperature and standard pressure and is introduced into the apparatus **1000**.

14. Method according to any of Claims 1 to 13, in which the inert gas streams (**30**) in step (I) and in step (II) each independently have a temperature in the range from 200°C to 600°C.

15. Method according to any of Claims 1 to 14, in which the amine (**2**) is selected from the group consisting of isophoronediamine, hexamethylenediamine, bis(p-aminocyclohexyl)methane, tolylenediamine and diphenylmethanediamine.

## Revendications

1. Procédé d'exploitation d'une unité de phosgénation en phase gazeuse (**100**) pour la mise en réaction d'une amine (**2**) avec du phosgène (**1**) en excès stoechiométrique par rapport aux groupes amino primaires de l'amine (**2**) pour former l'isocyanate correspondant (**4**), l'unité de phosgénation en phase gazeuse (**100**) comprenant au moins :
(i) un dispositif **1000** pour la préparation d'un courant de phosgène gazeux (**10**), comprenant éventuellement une substance inerte (**3**) en plus du phosgène (**1**),
(ii) un dispositif **2000** pour la préparation d'un courant d'amine gazeux (**20**), comprenant éventuellement une substance inerte (**3**) en plus de l'amine (**2**),
(iii) une zone de mélange (**3100**) pour le mélange des courants (**10**) et (**20**), la zone de mélange étant reliée par des appareils (**1100**, **2100**) respectivement avec le dispositif **1000** et le dispositif **2000**,
(iv) une zone de réaction (**3200**) agencée en termes d'écoulement après la zone de mélange (**3100**) pour la mise en réaction ultérieure des courants (**10**) et (**20**) mélangés auparavant,
(v) une zone d'arrêt de la réaction (**4000**) agencée en termes d'écoulement après la zone de réaction (**3200**) pour la fin de la réaction,
et éventuellement
(vi) une partie de traitement (**5000**) comprenant des appareils pour la récupération et le recyclage du phosgène non réagi (**1"**) (**5100**) et des appareils pour l'obtention de l'isocyanate fabriqué sous forme pure (**5200**),
**caractérisé en ce que**
les étapes suivantes sont réalisées pour la mise hors service de l'unité de phosgénation en phase gazeuse (**100**) :
(I) la réduction du débit massique d'amine M'(**2**) à zéro, un courant de gaz inerte gazeux (**30**) étant maintenu au travers de l'appareil **2100**, de la zone de mélange (**3100**), de la zone de réaction (**3200**) et de la zone d'arrêt de la réaction (**4000**) ;
(II) uniquement après l'écoulement d'une durée qui correspond à au moins 10 fois le temps de séjour, de préférence à au moins 30 fois le temps de séjour, de manière particulièrement préférée à au moins 60 fois le temps de séjour, de manière tout particulièrement préférée à au moins 90 fois le temps de séjour du phosgène (**1**) depuis la sortie du dispositif **1000** jusqu'à la sortie de la zone d'arrêt de la réaction (**4000**) en mode normal de l'unité de phosgénation en phase gazeuse (**100**), calculée à partir du moment à partir duquel M'(**2**) est de zéro :
la réduction du débit massique de phosgène M'(**1**) qui quitte le dispositif **1000** à zéro, un courant de gaz inerte gazeux (**30**) étant maintenu au travers de l'appareil **1100,** de la zone de mélange (**3100**), de la zone de réaction (**3200**) et de la zone d'arrêt de la réaction (**4000**) ;
(III) le maintien d'au moins le courant de gaz inerte (**30**) de (I), de préférence des courants de gaz inerte (**30**) de (I) et (II), au moins pendant une durée qui correspond à trois fois, de préférence à 100 fois, de manière particulièrement préférée à 200 fois, de manière tout particulièrement préférée à 350 fois, le temps de séjour du courant de gaz inerte (**30**) depuis l'entrée dans la zone de mélange (**3100**) jusqu'à la sortie de la zone d'arrêt de la réaction (**4000**), calculée à partir du moment à partir duquel le débit massique de phosgène M'(**1**) qui quitte le dispositif **1000** est de zéro.

2. Procédé selon la revendication 1, selon lequel l'unité de phosgénation en phase gazeuse (**100**) comprend la partie de traitement (**5000**) et selon lequel, en mode normal de l'unité de phosgénation en phase gazeuse (**100**), le phosgène (**1**) dans le courant gazeux de phosgène (**10**) est un mélange de phosgène frais (**1'**) et de phosgène recyclé (**1"**) récupéré dans les appareils **5100.**

3. Procédé selon la revendication 2, selon lequel, après la réduction du débit massique d'amine M'(**2**) à zéro,
(II.a) l'alimentation de phosgène frais (**1'**) est tout d'abord interrompue, puis
(II.b) uniquement après l'écoulement d'une durée qui correspond à au moins 10 fois le temps de séjour, de préférence à au moins 30 fois le temps de séjour, de manière particulièrement préférée à au moins 60 fois le temps de séjour, de manière tout particulièrement préférée à 90 fois le temps de séjour du phosgène (**1**) depuis la sortie du dispositif **1000** jusqu'à la sortie de la zone d'arrêt de la réaction (**4000**) en mode normal de l'unité de phosgénation en phase gazeuse (**100**), calculée à partir du moment à partir duquel M'(**2**) est de zéro, l'alimentation de phosgène recyclé (**1"**) est également interrompue, de sorte que le débit massique de phosgène M'(**1**) qui quitte le dispositif **1000** soit de zéro,
un courant de gaz inerte gazeux (**30**) étant maintenu au travers de l'appareil **1100,** de la zone de mélange (**3100**), de la zone de réaction (**3200**) et de la zone d'arrêt de la réaction (**4000**) pendant (II.a) et (II.b).

4. Procédé selon l'une quelconque des revendications 1 à 3, selon lequel les compositions et les débits massiques des courants **10** et **20** lors de l'exploitation de l'unité de phosgénation en phase gazeuse (**100**) en mode normal sont accordés les uns aux autres, de sorte que le phosgène (**1**) soit présent dans le courant **10** en un excès stoechiométrique par rapport aux groupes amino primaires de l'amine (**2**) dans le courant **20** d'au moins 150 % de la théorie.

5. Procédé selon l'une quelconque des revendications 1 à 4, selon lequel, à l'étape (I), le débit massique d'amine M'(**2**) est réduit à zéro de manière continue ou par paliers, de préférence de manière continue.

6. Procédé selon l'une quelconque des revendications 2 à 5, selon lequel, à l'étape (II.a), l'alimentation du phosgène frais (**1'**) est réduite à zéro de manière continue ou par paliers, de préférence de manière continue.

7. Procédé selon l'une quelconque des revendications 1 à 6, selon lequel l'appareil **1100** comprend une buse.

8. Procédé selon l'une quelconque des revendications 1 à 7, selon lequel l'appareil **2100** comprend une buse.

9. Procédé selon l'une quelconque des revendications 1 à 6, selon lequel les appareils **1100** et **2100** comprennent un dispositif à buse commun.

10. Procédé selon l'une quelconque des revendications 1 à 9, selon lequel la zone d'arrêt de la réaction (**4000**) est exploitée par mise en contact du produit de procédé gazeux sortant de la zone de réaction **(3200)** avec un solvant inerte en dessous de la température d'ébullition de l'isocyanate (**4**) et au-dessus de la température de décomposition du chlorure de carbamoyle correspondant à l'amine (**2**).

11. Procédé selon la revendication 10, selon lequel la zone d'arrêt de la réaction (**4000**) est mise hors service au plus tôt lorsque le débit massique de phosgène M'(**1**) qui quitte le dispositif **1000** est de zéro.

12. Procédé selon l'une quelconque des revendications 1 à 11, selon lequel le courant de gaz inerte (**30**) est introduit à l'étape (I) dans le dispositif **2000** ou dans l'appareil **2100,** et selon lequel le courant de gaz inerte (**30**) est introduit à l'étape (II) dans le dispositif **1000** ou l'appareil **1100.**

13. Procédé selon l'une quelconque des revendications 1 à 11, selon lequel le courant de gaz inerte (**30**) à l'étape (I) est obtenu par chauffage d'une substance inerte (**3**) introduite dans le dispositif **2000,** qui est de préférence liquide à température ambiante et pression normale, et selon lequel le courant de gaz inerte (**30**) à l'étape (II) est obtenu par chauffage d'une substance inerte (**3**) introduite dans le dispositif **1000,** qui est de préférence liquide à température ambiante et pression normale.

14. Procédé selon l'une quelconque des revendications 1 à 13, selon lequel les courants de gaz inerte (**30**) à l'étape (I) et à l'étape (II) présentent indépendamment l'un de l'autre une température dans la plage allant de 200 °C à 600 °C.

15. Procédé selon l'une quelconque des revendications 1 à 14, selon lequel l'amine (**2**) est choisie dans le groupe constitué par l'isophorone-diamine, l'hexaméthylène-diamine, le bis(p-aminocyclohexyl)méthane, la toluylène-diamine et la diphénylméthane-diamine.
